# EUROPEAN PATENT SPECIFICATION

(11) **EP 4 117 632 B1**
(45) Date of publication and mention of the grant of the patent: **21.01.2026**
(21) Application number: 21710016.3
(22) Date of filing: 11.03.2021
(51) Int. Cl.: A61K 9/107, A61K 47/12, A61K 47/24

(54) **SUNFLOWER PHOSPHOLIPID COMPOSITION CONTAINING PHOSPHATIDYLCHOLINE**
SONNENBLUMENPHOSPHOLIPIDZUSAMMENSETZUNG MIT PHOSPHATIDYLCHOLIN
COMPOSITION DE PHOSPHOLIPIDES DE TOURNESOL CONTENANT DE LA PHOSPHATIDYLCHOLINE

(30) Priority: 12.03.2020 EP 20162785
(43) Date of publication of application: 18.01.2023
(73) Proprietor: Lipoid GmbH, 67065 Ludwigshafen (DE)
(72) Inventor: VAN HOOGEVEST, Peter, 67065 Ludwigshafen (DE); HEIDECKE, Christoph, 67065 Ludwigshafen (DE)
(74) Representative: Meissner Bolte Partnerschaft mbB
(86) International application number: PCT/EP2021/056187
(87) International publication number: WO 2021/180848

(56) References cited:
- EP-A1- 2 857 042
- LI LIANG ET AL: "Physical and Oxidative Stability of Flaxseed Oil-in-Water Emulsions Fabricated from Sunflower Lecithins: Impact of Blending Lecithins with Different Phospholipid Profiles", JOURNAL OF AGRICULTURAL AND FOOD CHEMISTRY, vol. 65, no. 23, 14 June 2017 (2017-06-14), pages 4755 - 4765, XP055729021, ISSN: 0021-8561, DOI: 10.1021/acs.jafc.7b01469
- DARIO M. CABEZAS ET AL: "Emulsifying properties of hydrolysed and low HLB sunflower lecithin mixtures : Hydrolysed sunflower lecithin mixed with low HLB sunflower lecithin", EUROPEAN JOURNAL OF LIPID SCIENCE AND TECHNOLOGY., vol. 118, no. 7, 9 October 2015 (2015-10-09), DE, pages 975 - 983, XP055467850, ISSN: 1438-7697, DOI: 10.1002/ejlt.201500182
- "Food Industry", 16 January 2013, INTECH, ISBN: 978-953-51-0911-2, article DARIO M. ET AL: "Antioxidant and Emulsifying Properties of Modified Sunflower Lecithin by Fractionation with Ethanol-Water Mixtures", XP055730114, DOI: 10.5772/53165
- ROSELENA S. SCHUH ET AL: "PHYSICOCHEMICAL PROPERTIES OF LECITHIN-BASED NANOEMULSIONS OBTAINED BY SPONTANEOUS EMULSIFICATION OR HIGH-PRESSURE HOMOGENIZATION", QUIMICA NOVA, 1 January 2014 (2014-01-01), BR, XP055730561, ISSN: 0100-4042, DOI: 10.5935/0100-4042.20140186

## Description

The present invention relates to sunflower phospholipid compositions, i.e. phospholipids obtained from sunflower, and to their use as emulsifiers in aqueous oil-in-water emulsions for use in the parenteral administration. The present invention also relates to aqueous oil-in-water emulsions containing such sunflower phospholipid compositions and a method for their production.

Oil-in-water-emulsions for parenteral application or administration, also termed parenteral fat emulsions, are sterile oil-in-water-emulsions of physiologically tolerated oils, which are formulated in such a way that they are suitable for parenteral administration - for the requirements of parenteral fat emulsions see e.g. Driscoll, D., Lipid Injectable Emulsions: Pharmacopeial and Safety Issues, Pharmaceutical research 2006, 23, 1959-69. 10.1007/s11095-006-9092-4; Driscoll, D., Journal of Parenteral and Enteral Nutrition 2017, 41, 125- 134, Driscoll, D., Commercial Lipid Emulsions and All-in-One-Mixtures for Intravenous Infusion - Composition and Physicochemical Properties, in "Intravenous Lipid Emulsions" (Calder, P.C. et al, Eds), Karger Medical and Scientific Publishers 2014, pp. 48 - 56 and the references cited therein; Calder P.C. et al, Intensive Care Medicine, 2010, 36(5), 735-749.

EP2857042A1 describes a sterile, ready-to-use, pharmaceutical oil-in-water emulsion composition for parenteral administration comprising: an oil, an aqueous phase, a progestogen, and a phospholipid, wherein the progestogen:oil (wt./wt.) ratio is at least 1:32, wherein the composition contains less than 1.5% wt./wt. polyethylene glycol 15-hydroxystearate; and wherein the composition contains less than 2.5% (wt./vol.) benzyl benzoate.

Oil-in-water emulsions of physiologically tolerated oils are mainly used for parenteral nutrition. For parenteral nutrition, patients are fed with all necessary nutrients, such as carbohydrates, fat, vitamins, electrolytes, trace elements and water, by injectable liquid formulations, which are administered intravenously, i.e. into the veins of a patient in need of a parenteral nutrition. Fat is an essential part of total parenteral nutrition, because it delivers essential fatty acids to the body of the patient that cannot be synthesized by the body itself. Parenteral nutrition without fat leads to essential fatty acid deficiency in a couple of days. Since fat is immiscible with blood, it can only be administered parenterally in the form of an aqueous emulsion, because direct injection of fat would result in blocking of blood vessels. For a review of parenteral nutrition see e.g. M. Stawny et al., Pharmaceutical Point of View on Parenteral Nutrition, The Scientific World Journal 2013: https://doi.org/10.1155/2013/415310, and the references cited therein; Driscoll, D. loc.cit.

Apart from parenteral nutrition, parenteral fat emulsions may be used for parenteral drug delivery of lipophilic drugs. For this, the lipophilic drug is dissolved in the oil phase of the parenteral fat emulsion. Formulation of drugs in parenteral fat emulsions have numerous advantages compared to other formulations, such as reduction of pain and irritation at the injection site, thrombophlebitis, reduced drug toxicity, enhanced drug stability, and the possibility for targeted drug delivery - see e.g. Hippalgoankar K, Majumdar S, Kansara V, Injectable Lipid Emulsions - Advancements, Opportunities and Challenges, AAPS PharmSciTech 2010, 11:4, 1526-1540. Typical examples of drugs formulated in parenteral fat emulsions are propofol, aprepitant, diazepam, etomidate and clevidipine butyrate.

Recently, parenteral fat emulsions have been suggested for the treatment of drug overdose or intoxication with lipophilic drugs. By intravenous injection of the parenteral fat emulsion to a patient suffering from drug overdose or intoxication, the lipophilic drug is extracted into the emulsion phase of the injected fat emulsion - see e.g. Mirtallo et al., The Annals Pharmacotherapy, 2010, 44; Rothschild et al., Scandinavian Journal of Trauma, Resuscitation and Emergency Medicine 2010, 18, 51-58.

Oil-in-water-emulsions are heterogeneous systems consisting of an oily phase homogeneously dispersed or emulsified in an aqueous phase. As they are thermodynamically unstable systems, an emulsifier is required to prevent the coalescence of the oil droplets thereby stabilizing the emulsion. While synthetic emulsifiers, such as poloxamers, macrogol-hydroxystearates or polysorbates, may be used for stabilizing parenteral fat emulsions, phospholipids are preferred emulsifiers for this kind of application because of their excellent toxicity profile. Toxicity is a very important aspect in the choice of the emulsifier for parenteral fat emulsions because of the high amounts of emulsifier administered during parenteral nutrition. For example, for a person with a body weight of 70 kg, up to 21 g of phospholipids are administered per day. Since phospholipids are natural constituents of the human body's cell membranes and metabolized in the same way as other nutritional fats, they can be administered in very high doses without any reported negative side effects. In contrast, unwanted side effects and toxicity, such as fat overload syndrome, hypersensitivity and infusion site adverse reactions, have been reported for parenteral emulsions and other injectables containing synthetic emulsifiers. For this reason, it is desirable to formulate parenteral fat emulsions without the use of synthetic emulsifiers.

Phospholipids are natural products having an amphiphilic character. The structure of the phospholipid molecule generally consists of a fatty acid diglyceride moiety, which is esterified with a phosphate group. The phosphate groups can be further modified with simple organic molecules such as choline, glycerol, ethanolamine, inositol or serine resulting in different phospholipid compounds. Common sources of phospholipids are soya, rapeseed, sunflower, chicken eggs, bovine milk, fish eggs, etc. Each source has a unique profile of individual phospholipid compounds in the phospholipid and with regard to the fatty acid pattern and consequently differing applications in food, nutrition, pharmaceuticals, cosmetics and drug delivery.

For parenteral fat emulsions, typically egg phospholipids, i.e. phospholipids obtained from chicken egg, are the state of the art emulsifier. In most cases, egg phospholipids with a phosphatidylcholine content of ca. at least 70 to 80% are used. Phosphatidylcholine is a zwitterionic compound in the pH range of 6 to 8 usually required in parenteral fat emulsions.

Despite the egg phospholipids being a very effective emulsifier for fat emulsion, there are some drawbacks of egg phospholipids:
- Potential allergenicity, which is caused by the presence of residual egg protein in the phospholipid.
- Egg phospholipids are an animal derived product, which may give rise to consumer concerns, cause environmental impact and increase the risk for possibly transmittable avian diseases.

L. Liang et al., J. Agricultural and Food Chemistry, 65 (23) (2017), S. 4755-4765 describe the physicochemical and oxidative stability of linseed o/w emulsions produced using sunflower lecithin. Specifically, Sunlipon 50 and Sunlipon 90 were used. Sunlipon 50 contains 58 wt.% phosphatidylcholine, 1 wt.% phosphatidylglycerol and 0.2 wt.% phosphatidic acid.

D. M. Cabezas et al., European J. of Lipid Science and Technology, 118 (7) (2015), S. 975-983 disclose a study on three modified sunflower lecithins (hydrolysed, HL), deoiled sunflower lecithins (DSL), and PI fraction (Plf) and their o/w emulsifying properties of modified lecithins with low HLB mixing them with hydrolysed sunflower lecithin. Dioled sunflower lecithin prepared by acetone extraction, followed by ethanol fraction, then applied as emulsifying agent of coarse and fine o/w emulsions. Stability of different emulsions was evaluated. Mistures with a hydrolysed lecithin increased the HLB (addition of lysophospholipids) and improved the o/w emulsifying properties of DSL and Plf in each case. There is no content of chlorogenic acid mentioned therein.

D. M. Cabezas et al., Food Industry 2013, S. 589-602 disclose antioxidant and emulsifying properties of modified sunflower lecithin by fractionation with ethanol-water mixtures, by using different modificationsprocesses, such as deoiling, ethanol fractionation, modified lecithins were obtained with better physiochemical and functional properties, e.g. improved antioxidant activity, oxidative stability. There is no content of chlorogenic acid mentioned therein.

R. S. Schuh et al., Quimica Nova 2014, S. 1196 disclose the feasibility of preparing injectable lipid emulsions composed of sunflower lecithins by spontaneous emulsification an high-pressure homogenization, as alternatives to traditional egg-lecithin nanoemulsions for patients who are sensitive to egg derivatives. There is no content of chlorogenic acid mentioned therein.

Although there have been several attempts to replace egg phospholipids in commercial fat emulsions by plant based phospholipids, such as soybean phospholipids or rape seed phospholipids, either alone or in combination with synthetic emulsifiers of the poloxamer type, these products had other drawbacks and have meanwhile been withdrawn from the market.

Therefore, there is still a need for emulsifiers for parenteral fat emulsions, which have low toxicity and do provide for good emulsion stability. Moreover, the emulsifiers should be compatible with alkaline pH values of pH 7 or higher usually encountered in the production of parenteral fat emulsions, in particular those for parenteral nutrition and should not result in a discoloration of the emulsion.

It was now found that phospholipid compositions obtained from sunflower seeds, hereinafter also termed sunflower phospholipids and sunflower phospholipid compositions which contain phosphatidylcholine efficiently stabilize parenteral fat emulsions and minimize discoloration if they contain less than 0.1% by weight of chlorogenic acid and are combined with one or more anionic phospholipid compounds. Thus these combinations can be used as an emulsifier in parenteral fat emulsions. Moreover, they do not present an allergenic risk or a toxicity issue.

Therefore, a first aspect of the present invention relates to a sunflower phospholipid composition containing phosphatidylcholine and containing not more than 0.1% by weight, in particular not more than 0.08% by weight or not more than 0.05% by weight and especially not more than 0.02% by weight of chlorogenic acids, based on the total weight of the sunflower phospholipid composition, in combination with at least one anionic stabilizer compound comprising at least one anionic phospholipid compound as an emulsifier in an aqueous oil-in-water emulsion for use in the parenteral administration, herein abbreviated as parenteral fat emulsion.

Any references in the description to methods of treatment refer to the compounds, pharmaceutical compositions and medicaments of the present invention for use in a method for treatment of the human (or animal) body by therapy (or for diagnosis).

The combination of sunflower compositions and at least one anionic stabilizer compound comprising at least one anionic phospholipid compound are useful as emulsifiers and provide particular benefits to the parenteral fat emulsions. In particular, sunflower phospholipid compositions containing a sufficient amount of phosphatidylcholine of e.g. 45% by weight or higher, e.g. from 45 to 99% by weight, in particular from 50 to 98.5% by weight, especially from 60 to 98% by weight or 70 to 98% by weight, based on the total weight of the sunflower phospholipid composition, and having a low content of chlorogenic acids of less than 0.1% by weight, in particular not more or less than 0.08% by weight or not more or less than 0.05% by weight and especially not more or less than 0.02% by weight, provide parenteral fat emulsions showing reduced or even no discoloration during production or storage. Due to the low content of chlorogenic acid they also show better compatibility with compounds having amino groups such as amino acids, as the presence of chlorogenic acid may cause severe discoloration during production or storage when these compounds are present in parenteral fat emulsions. These sunflower phospholipid compositions containing a sufficient amount of phosphatidylcholine are combined with one or more anionic stabilizer compound comprising at least one anionic phospholipid, the combination imparts improved stability to aqueous oil-in-water emulsions against coalescence of the oil-droplets and also minimizes discoloration of the emulsions and thus the combinations provides improved long-term storage stability for several month and up to years.

Therefore, a second aspect of the present invention relates to combinations of sunflower phospholipid compositions containing phosphatidylcholine in an amount of at least 45% by weight, e.g. from 45 to 99% by weight, in particular from 50 to 98.5% by weight, especially from 60 to 98% by weight or 70 to 98% by weight, based on the total weight of the sunflower phospholipid composition, and having a content of chlorogenic acids of less than 0.1% by weight, in particular not more or less than 0.08% by weight or not more or less than 0.05% by weight and especially not more or less than 0.02% by weight, based on the total weight of the sunflower phospholipid composition with one or more anionic stabilizer compounds comprising at least one anionic phospholipid.

The inventors also found that the combinations can be formulated as a single composition. Therefore a third aspect of the present invention relates to sunflower phospholipid compositions containing phosphatidylcholine in an amount of at least 45% by weight, e.g. from 45 to 98.8% by weight, in particular from 50 to 98.5% by weight, especially from 60 to 98% by weight or from 70 to 98% by weight, based on the total weight of the sunflower phospholipid composition, and at least one anionic stabilizer compound comprising at least one anionic phospholipid compound and having a content of chlorogenic acids of less than 0.1% by weight, in particular not more or less than 0.08% by weight or not more or less than 0.05% by weight and especially not more or less than 0.02% by weight, based on the total weight of the sunflower phospholipid composition. The phospholipid compositions according to the third aspect of the present invention are hereinafter also termed "mixtures".

In a fourth aspect, the present invention relates to methods for producing the sunflower phospholipid compositions according to the third aspect of the present invention.

A fifth aspect of the present invention relates to aqueous oil-in-water emulsion for parenteral administration, in particular for parenteral nutrition, which contains
a) an oil phase comprising a triglyceride composition suitable for parenteral administration, in particular for parenteral nutrition,
b) a combination of a sunflower phospholipid composition having a content of chlorogenic acids of less than 0.1% by weight, in particular in particular not more or less than 0.08% by weight or not more or less than 0.05% by weight, especially not more or less than 0.02% by weight of chlorogenic acids, based on the total weight of the sunflower phospholipid composition, with at least one anionic stabilizer compound comprising at least one anionic phospholipid compound as defined herein or a mixture according to the third aspect of the invention as defined herein, and
c) water.

A sixth aspect of the present invention relates to a process for the production of such an emulsion.

Here and throughout the application, the technical terms have their common technical meanings, if not stated otherwise.

The term "sunflower phospholipid composition" and "sunflower lecithin" refers to phospholipid compositions and lecithin, respectively, which are obtained from sunflower oil. Usually, a sunflower phospholipid composition is obtained from a sunflower lecithin by fractionation while the sunflower lecithin is obtained from the dried sludge obtained in the degumming step of sunflower oil.

The term "phospholipid composition" refers to a composition of phospholipid molecules, wherein the total amount of phospholipid molecules in the composition is generally at least 80% by weight, frequently at least 90% by weight, the remainder being essentially triglycerides and/or fatty acids.

A sunflower phospholipid composition is distinct from the phospholipid compositions of other natural sources by their characteristic fatty acid composition - for the lecithins see e.g. Hoogevest, P. and Wendel, A., "The use of natural and synthetic phospholipids as pharmaceutical excipients." European journal of lipid science and technology 116.9 (2014): 1088-1107, in particular table 2 on page 1090, and Guiotto, E., Tomás, M., Diehl, B. (2015). Sunflower Lecithin. 10.1016/B978-1-63067-044-3.50007-8. Chapter 3, page 59, table 3.B. In particular, a sunflower phospholipid composition is distinct from the phospholipid compositions of other natural sources by its high content of linoleic acid, which is typically at least 60%, e.g. in the range of 60 to 75% and its low content of stearic acid, which is typically at most 8%, e.g. 1 to 8%. Typically the content of palmitic acid is at most 12%, e.g. in the range of 6 to 12%, and the oleic acid content is typically in the range of 12 to 25%. The % values given here refer to the relative peak area of the respective fatty acid in the sunflower phospholipid composition as determined by analytic HPLC. Consequently, the phospholipid compounds contained in such a sunflower phospholipid composition, such as phosphatidylcholine, phosphatidylethanolamine, N-acylphosphatidylethanolamine, and lysophosphatidylcholine and also any further phospholipid compound and fatty acid esters contained therein, such as fatty acid triglycerides, will have a similar fatty acid composition. Apart from that, the sunflower phospholipid compositions are distinct from the phospholipid compositions of other sources by the presence of characteristic residual sunflower protein, which in contrast to residual egg protein does not result in an allergenic risk.

According to the invention, the sunflower phospholipid compositions contain a phosphatidylcholine, which mainly serves for the stabilization of the oil-in-water emulsion, to be more precise, for the stabilization of the oil droplets of the oil in water emulsions. Usually, phosphatidylcholine is the main component of the sunflower phospholipid compositions. Preferably, the content of phosphatidylcholine in the sunflower phospholipid composition is at least 45.0% by weight, in particular at least 50.0% by weight, especially at least 60.0% by weight or at least 70% by weight, based on the total weight of the sunflower phospholipid composition. Frequently, the content of phosphatidylcholine will not exceed 99.0% by weight, in particular 98.5% by weight, especially 98.0% by weight, based on the total weight of the sunflower phospholipid composition.

Depending on the amount of phosphatidylcholine, the sunflower phospholipid compositions may contain one or more further phospholipid compounds different from phosphatidylcholine, in particular one or more of the following phospholipid compounds: phosphatidylethanolamine, N-acylphosphatidylethanolamine and lysophosphatidylcholine. The total amount of phospholipid compounds different from phosphatidylcholine is typically in the range of 1.0 to 50.0% by weight, in particular in the range of 1.5 to 45.0% by weight, especially in the range of 2.0 to 40.0% by weight or in the range of 2 to 30% by weight, based on the total weight of the sunflower phospholipid composition. For example, the amount of lysophosphatidylcholine may be from traces up to 25.0% by weight, based on the total weight of the sunflower phospholipid composition. Likewise, the amount of phosphatidylethanolamine may be from traces up to 25.0% by weight, based on the total weight of the sunflower phospholipid composition and is frequently in the range of 1.0 to 20.0% by weight. Typically, the amount of N-acylphosphatidylethanolamine will not exceed 10.0% by weight and may be present from traces up to 10.0% by weight, based on the total weight of the sunflower phospholipid composition. In a particular group of embodiments, the sunflower phospholipid compositions contain an anionic phospholipid compound, such as phosphatidylglycerol, for example in an amount in the range of 0.1 to 2% by weight, based on the total weight of the sunflower phospholipid composition.

The sunflower phospholipid composition may contain lysophosphatidylcholine. For pharmacological reasons, the amount of lysophosphatidylcholine does preferably not exceed 10% by weight and is in particular not more than 5% by weight, e.g. in the range of 0.1 to 5.0% by weight, based on the total weight of the sunflower phospholipid composition.

For the purpose of the invention, it has been found beneficial, when the sunflower phospholipid composition contains phosphatidylethanolamine, in particular in an amount in the range of 1.0 to 25.0% by weight, frequently in the range of 2.0 to 20.0% by weight, based on the total weight of the sunflower phospholipid composition.

Furthermore, the sunflower phospholipid compositions may contain one or more fatty acid triglycerides and/or free fatty acids, which are residues from their production process. Typically, the total amount of triglycerides and free fatty acids will not exceed 10.0% by weight, based on the total weight of the sunflower phospholipid composition.

Due to the presence of free fatty acids, the sunflower phospholipid compositions of the present inventions may have an acid number different from 0. Here and in the following, the acid number refers to the acid number in mg KOH / g. Usually, the acid number will not exceed a value of 50, in particular 40 and is frequently in the range of 0.01 to 35.

According to a particular group 1 of embodiments, the sunflower phospholipid compositions have a content of phosphatidylcholine of at least 85.0% by weight, in particular at least 90.0% by weight, e.g. from 85.0 to 99.0% by weight, in particular from 90.0 to 98.5% by weight, based on the total weight of the sunflower phospholipid composition. In these sunflower phospholipid compositions, the total amount of phospholipid compounds different from phosphatidylcholine is typically in the range of 1.0 to 12.0% by weight, in particular in the range of 1.5 to 10.0% by weight, based on the total weight of the sunflower phospholipid composition. For example, the amount of lysophosphatidylcholine may be from traces up to 5.0% by weight, based on the total weight of the sunflower phospholipid composition. Likewise, the amount of phosphatidylethanolamine may be from traces up to 1.0% by weight, based on the total weight of the sunflower phospholipid composition. Typically, the amount of N-acylphosphatidylethanolamine will not exceed 1.0% by weight, based on the total weight of the sunflower phospholipid composition. The amount of free fatty acids and fatty acid triglycerides if frequently below 5% by weight, based on the total weight of said sunflower phospholipid compositions. The acid number of these sunflower phospholipid compositions is typically below 5.

According to another particular group 2 of embodiments, the sunflower phospholipid compositions have a content of phosphatidylcholine in the range of 45 to 85.0% by weight, in particular in the range of 50 to 85.0% by weight, based on the total weight of the sunflower phospholipid composition. In these sunflower phospholipid compositions, the total amount of phospholipid compounds different from phosphatidylcholine is typically in the range of 1.0 to 50.0% by weight, in particular in the range of 1.5 to 45.0% by weight, based on the total weight of the sunflower phospholipid composition. For example, the amount of lysophosphatidylcholine may be from 1.0 to 25.0% by weight, frequently in the range of 2.0 to 20.0% by weight, based on the total weight of the sunflower phospholipid composition. Likewise, the amount of phosphatidylethanolamine may be in the range of 1.0 to 25.0% by weight, based on the total weight of the sunflower phospholipid composition and is frequently in the range of 2.0 to 20.0% by weight. Typically, the amount of N-acylphosphatidylethanolamine will not exceed 10.0% by weight and may be present from traces up to 10.0% by weight, based on the total weight of the sunflower phospholipid composition. The amount of free fatty acids and fatty acid triglycerides if frequently below 10% by weight, based on the total weight of said sunflower phospholipid compositions. The acid number of these sunflower phospholipid compositions is typically at most 50, e.g. in the range of 2 to 50.

Due to the presence of unsaturated fatty acids present in the phospholipid compounds of the sunflower phospholipid compositions, the sunflower phospholipid compositions will typically show an iodine number of at least 80, in particular at least 85. Frequently, the iodine number of the sunflower phospholipid compositions will not exceed 130 and is especially in the range of 85 to 120.

For the purpose of the invention, the sunflower phospholipid composition is generally used in such an amount that the concentration of phosphatidylcholine in the oil-in-water emulsion is in the range of 0.1 to 20% by weight, in particular in the range of 0.2 to 5% by weight, especially in the range of 0.3 to 3% by weight, based on the total weight of the water-in-oil emulsion. In particular, the amount of the sunflower phospholipid composition is chosen such that the weight ratio of phosphatidylcholine and oil phase in the oil-in-water emulsion is in the range of 0.5 : 95 to 1 : 3, in particular in the range of 1 : 99 to 1 : 5 and especially in the range of 2 : 98 to 1 : 8.

The sunflower phospholipid compositions according to the present invention and in particular the sunflower phospholipid compositions according to groups 1 and 2 of embodiments may be prepared by analogy to the processes for producing other plant-oil derived phospholipid compositions, such as phospholipids from soybean or rapeseed. A review on these processes is given by van Hoogevest, P. and Wendel, A., "The use of natural and synthetic phospholipids as pharmaceutical excipients." European journal of lipid science and technology 116.9 (2014): 1088-1107. Further details can be taken from the references cited therein. For example, a sunflower lecithin fraction obtained from the degumming step of sunflower oil is deoiled to obtain a solid lecithin product, which is then fractionated into fractions with higher phosphatidylcholine content, e.g. by single or multistep ethanolic extraction, by chromatographic purification and or combinations of ethanolic extraction and chromatographic purification, especially for producing sunflower phospholipid compositions having a content of phosphatidylcholine of at least 70% by weight or at least 80% by weight.

As pointed out above, the inventors of the present invention found that the presence of noticeable chlorogenic acids in the sunflower phospholipid compositions used for parenteral fat emulsions cause an undesirable discoloration of the parenteral fat emulsions, presumably because chlorogenic acids tend to form green pigments at pH values of pH > 6, in particular at pH 7 or higher, usually encountered in the production of parenteral fat emulsions. Moreover, parenteral fat emulsions tend to be less stable if the sunflower phospholipid compositions used for their production contain noticeable amounts of chlorogenic acids. Therefore, the sunflower phospholipid compositions and in particular the sunflower phospholipid compositions according to groups 1 and 2 of embodiments contain less than 0.1% by weight, based on the weight of the respective sunflower phospholipid composition. In particular, the sunflower phospholipid compositions contain at most 0.08% by weight, especially at most 0.05% by weight of chlorogenic acids, based on the total weight of the sunflower phospholipid composition.

The term "chlorogenic acids" as used herein refers to the compound chlorogenic acid, i.e. (1S,3R,4R,5R)-3-[(E)-3-(3,4-dihydroxyphenyl)prop-2-enoyl]oxy-1,4,5-trihydroxycyclohexane-1-carboxylic acid, and related esters of polyphenol compounds with quinic acid (1,4,5-trihydroxycyclohexane-1-carboxylic acid), namely esters of hydroxycinnamic acid compounds such as caffeic acid, ferulic acid and p-coumaric acid.

The amounts of chlorogenic acids given herein therefore refer to the total amount of such chlorogenic acid compounds in the sunflower phospholipid composition.

Sunflower phospholipid compositions and in particular the sunflower phospholipid compositions according to groups 1 and 2 of embodiments, which contain less than 0.1% by weight, in particular not more than 0.08% by weight or not more than 0.05% by weight, especially not more than 0.02% by weight of chlorogenic acids, based on the total weight of the sunflower phospholipid composition, can be prepared by a process which comprises the following steps:
i) extracting a sunflower lecithin, in particular a de-oiled sunflower lecithin, with a solvent selected from acetone and mixtures thereof with water, to obtain an extract and a residue, followed by
ii) repeated extraction of the residue with ethanol or a mixture thereof with water to obtain ethanolic extracts,
iii) combining the ethanolic extracts and removing the ethanol and optional water to obtain sunflower phospholipid composition containing phosphatidylcholine having a content of chlorogenic acids of less than 0.1% by weight, in particular at most 0.08% by weight, especially at most 0.05% by weight, based on the total weight of the sunflower phospholipid composition.

In step i. the major portion of chlorogenic acid will be removed from the sunflower lecithin. The sunflower lecithin is in particular a de-oiled sunflower lecithin but a not yet de-oiled sunflower lecithin may be used in step i. instead. In step i. the solvent is preferably a mixture acetone and water, in particular a mixture of acetone and water, wherein the weight ratio of acetone to water is from 1 : 9 to 4 : 1.

Depending on the amount of water in the ethanolic solvent used in step ii. the extract will be enriched with regard to phosphatidylcholine. The solvent in step ii. is preferably ethanol having a water content of at most 20% by weight, in particular at most 10% by weight.

In step iii. a sunflower phospholipid composition which contains phosphatidylcholine usually in an amount of at least 45% by weight, in particular at least 50% by weight, e.g. from 45 to 99% by weight, in particular from 50 to 98.5% by weight, based on the total weight of the sunflower phospholipid composition is obtained.

Subsequent to step iii. a further chromatographic purification may follow, whereby the content of phosphatidylcholine will be further increased e.g. to a value of at least 85% by weight or at least 90% by weight, depending from the desired grade of the sunflower phospholipid composition.

For the stability of parenteral fat emulsions, it is favorable if the sunflower phospholipid compositions are used in combination with at least one anionic stabilizer compound comprising at least one anionic phospholipid compound. Anionic stabilizer compounds, which are also termed "anionic stabilizers", are anionic compounds which bear at least one fatty acid group and which in water at a concentration of 1% by weight and a pH value of pH 6 bear a negative charge. By the use of an anionic stabilizer compound a negative charge is imparted to the oil droplets of the parenteral fat emulsion, thereby facilitating the emulsification process and stabilizing the emulsion by repulsive electrostatic forces. The use of these anionic stabilizer compounds results in a negative zeta potential on the oil droplets. In this regard, anionic phospholipids have been found particularly beneficial. The anionic phospholipids may be the sole anionic stabilizer compounds. However, they may be combined with one or more further anionic stabilizers or anionic stabilizer compounds, respectively, which are different from anionic phospholipids.

Examples of anionic stabilizer compounds other than anionic phospholipids are fatty acids.

The anionic stabilizer compounds may be used in their acidic form or in the form of a pharmaceutically acceptable salts thereof, e.g. in the form of their ammonium salts or sodium salts.

Fatty acids which are suitable as further anionic stabilizer compounds are saturated and unsaturated aliphatic monocarboxylic acids having 6 to 22 carbon atoms such as caprylic acid, capric acid, lauric acid, myristic acid, palmitic acid, stearic acid, arachidic acid, behenic acid, lignoceric acid, cerotic acid, myristoleic acid, sapienic acid, oleic acid, elaidic acid, vaccenic acid, linoleic acid, α-linolenic acid, eicosapentaenoic acid and erucic acid as well as mixtures thereof. The aliphatic monocarboxylic acids may be used in their acidic form or in the form of a pharmaceutically acceptable salt thereof, in particular in the form of the sodium salt.

According to the invention, the anionic stabilizer compounds comprise at least one anionic phospholipid compound. These anionic phospholipid compounds may be the sole anionic stabilizer compound but they may also be used in combination with a fatty acid. Examples of anionic phospholipid compounds are phosphatidylglycerol, phosphatidylinositol, PEGylated phosphatidylethanolamine phosphatidylserine, phosphatidic acid, 1,3-bis(sn-3'-phosphatidyl)-sn-glycerol, also termed cardiolipin, and combinations thereof. As mentioned before, the anionic phospholipid compounds may be used in their acidic form or in the form of a pharmaceutically acceptable salt thereof, in particular in the form of the sodium salt or the ammonium salt thereof.

PEGylated phosphatidylethanolamines are compounds based on phosphatidylethanolamine, where the amino group of the 2-aminoethoxy group bears a polyethyleneoxide (PEG) radical attached via a carbonyl group to the nitrogen atom. Such PEG radicals will generally have number average molecular weights in the range of 200 to 10000. These compounds have typically the following formula: 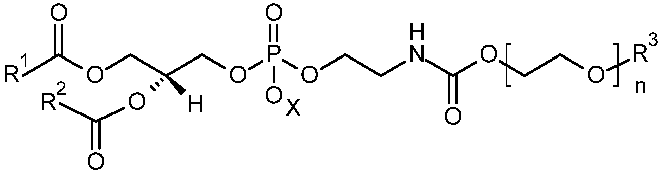 where C(O)-R¹ and C(O)-R² are fatty acid residues having typically from 10 to 20 carbon atoms and being saturated or unsaturated, such as steaoryl, palmitoleyl, myristoyl, or oleoyl and combinations thereof, R³ is hydrogen or alkyl, such as methyl, X is a physiologically acceptable cation, such as sodium or ammonium, and n is the number of oxyethylene repeating units, e.g. a number in the range of 4 to 250, corresponding to PEG radicals with number average molecular weights in the range of 200 to 10000.

The anionic phospholipid compounds are usually obtained from natural sources, in particular from sunflower oil. However, the synthetic analogues of these anionic phospholipid compounds are likewise suitable. The synthetic phospholipid analogues may contain one or two of the above mentioned fatty acids in the form of a diacylglyceryl group.

The anionic phospholipid compound may be the sole anionic stabilizer. However, it is also possible to use an anionic stabilizer which is a combination of at least one anionic phospholipid compound with at least one further anionic stabilizer compound, which is not an anionic phospholipid compound, in particular, in particular a fatty acid or fatty acid salt. In such combinations, the weight ratio of anionic phospholipid compound(s) to further anionic stabilizer compounds is in the range of 10 :1 to 1 : 10, in particular in the range of 8 : 1 to 1 : 3.

In the combination of the sunflower phospholipid composition with one or more anionic stabilizer compounds, the weight ratio of sunflower phospholipid composition to the total amount of anionic stabiliser is generally in the range of 99.8 : 0.2 to 80.0 : 20.0, frequently in the range of 99.8 : 0.2 to 90.0 : 10.0, in particular in the range of 99.5 : 0.5 to 91.0 : 9.0, preferably in the range of 99.0 : 1.0 to 92.0 : 8.0 and especially in the range of 98.5 : 1.5 to 93.0 : 7.0. In the combination of the invention, the weight ratio of sunflower phospholipid composition to the anionic phospholipid is generally in the range of 99.8 : 0.2 to 80.0 : 20.0, frequently in the range of 99.8 : 0.2 to 90.0 : 10.0, in particular in the range of 99.5 : 0.5 to 91.0 : 9.0, preferably in the range of 99.0 : 1.0 to 92.0 : 8.0 and especially in the range of 98.5 : 1.5 to 93.0 : 7.0. In the combination of the invention, the weight ratio of phosphatidylcholine to the anionic phospholipid is generally in the range of 99.8 : 0.2 to 70.0 : 30.0, frequently in the range of 99.8 : 0.2 to 85.0 : 15.0, in particular in the range of 99.5 : 0.5 to 90.0 : 10.0, especially in the range of 99.0 : 1.0 to 90.0 : 10.0 and especially in the range of 98.5 : 1.5 to 91.0 : 9.0.

A particularly preferred anionic stabilizer compound is phosphatidylglycerol. Suitable phosphatidyl glycerol may be obtained from animal or preferably from plant sources, such as phosphatidylglycerol from egg, sunflower oil, soy oil, rape seed oil, canola oil, palm oil or oat oil. Likewise, the suitable or even preferred are synthetic or semisynthetic phosphatidylglycerols like 1,2-dipalmitoyl-sn-glycero-3-phosphorylglycerol (DPPG), 1,2-dimyristoyl-sn-glycero-3-phosphorylglycerol (DMPG), 1,2-distearoyl-sn-glycero-3-phosphorylglycerol (DSPG), 1,2-distearoyl-sn-glycero-3-phosphorylglycerol (DOPG) and 1,2-palmitoyl/oleyl-sn-glycero-3-phosphorylglycerol (1-palmitoyl-2-oleoyl-sn-glycero-3-phosphorylgylcerol, 1-oleoyl-2-palmitoyl-sn-glycero-3-phosphorylgylcerol and mixtures thereof; POPG). The phosphatidylglycerol is in particular phosphatidylglycerol obtained from sunflower, especially 1,2-dimyristoyl-sn-glycero-3-phosphorylglycerol, which is also termed DMPG, or a pharmaceutically acceptable salt thereof, especially its sodium salt. Especially DMPG or DMPG prepared from glycerylphosphorylcholine, obtained from sunflower phospholipids is preferred. Likewise phosphatidylglycerol obtained from sunflower phosphatidylcholine by conversion using phospholipase D or a pharmaceutically acceptable salt thereof is suitable. If the sunflower phospholipid compositions are used in combination with phosphatidylglycerol, in particular 1,2-dimyristoyl-sn-glycero-3-phosphorylglycerol, or a pharmaceutically acceptable salt thereof, the weight ratio of sunflower phospholipid composition to phosphatidylglycerol, calculated as its acid form, is generally in the range of 99.8 : 0.2 to 80.0 : 20.0, frequently in the range of 99.8 : 0.2 to 90.0 : 10.0, in particular in the range of 99.5 : 0.5 to 91.0 : 9.0, preferably in the range of 99.0 : 1.0 to 92.0 : 8.0 and especially in the range of 98.5 : 1.5 to 93.0 : 7.0.

For the stability of parenteral fat emulsions and also for the reproducibility of the emulsification process and also for the homogenization process, it is favorable, when the combination of the sunflower phospholipid composition and the anionic stabilizer compound is used as a preformed mixture of the sunflower phospholipid composition and the anionic stabilizer compound. Without being bound to theory it is believed that the improved reproducibility of the emulsification/homogenization process is based on the following phenomenon. As phosphatidylcholine and anionic stabilizers, in particular anionic phospholipid compounds, spontaneously form liposomes when dispersed in water, the separate addition of phosphatidylcholine and anionic stabilizers to the aqueous phase will likely form stable liposomes of different composition with regard to the relative amounts of phosphatidylcholine and anionic stabilizer molecules contained therein. Since the exchange of phospholipid molecules between these liposomes is slow, the distribution of the different phospholipid molecules in the water/oil interface is inhomogeneous, which may cause instability of the emulsion and result in a poorer reproducibility of the emulsification/homogenization step. Using a preformed mixture of the phosphatidylcholine and the anionic stabilizer compound will significantly reduce or even largely avoid the formation of liposomes with different compositions and result in a more homogeneous distribution of the different phospholipid molecules in the water/oil interface and result in an improved reproducibility of the emulsification/homogenization step.

These mixtures contain at least one anionic stabilizer compound in addition to the phosphatidylcholine. In these mixtures the anionic stabilizer compound comprises at least one anionic phospholipid compound, in particular at least one anionic phospholipid compound, which is selected from the group consisting of phosphatidylglycerol, phosphatidylinositol, phosphatidylserine, phosphatidic acid, 1,3-bis(sn-3'-phosphatidyl)-sn-glycerol and combinations thereof. In these mixtures, the anionic stabilizer compound may be present in its free acid form or in the form of a pharmaceutically acceptable salt thereof. In these mixtures, the anionic stabilizer compound is even more preferably phosphatidylglycerol, in particular phosphatidylglycerol obtained from sunflower, especially DMPG, or a pharmaceutically acceptable salt thereof, especially its sodium salt. In these mixtures, the weight ratio of phosphatidylcholine to the total amount of anionic stabiliser is generally in the range of 99.8 : 0.2 to 70.0 : 30.0, frequently in the range of 99.8 : 0.2 to 85.0 : 15.0, in particular in the range of 99.5 : 0.5 to 90.0 : 10.0, preferably in the range of 99.0 : 1.0 to 90.0 : 10.0 and especially in the range of 98.5 : 1.5 to 91.0 : 9.0.

The aforementioned mixtures of a sunflower phospholipid composition and the at least one anionic stabilizer compound comprising at least one anionic phospholipid compound correspond to the sunflower phospholipid compositions of the third aspect of the present invention, if the amount of phosphatidylcholine contained therein is at least 45% by weight or higher, in particular at least 50.0% by weight, especially at least 55.0% by weight, based on the total weight of the mixture, e.g. from 45 to 98.8% by weight, in particular from 50 to 98.0% by weight, especially from 55 to 97.0% by weight, based on the total weight of the sunflower phospholipid composition. Depending on the amount of phosphatidylcholine, the sunflower phospholipid compositions of the third aspect of the invention may contain one or more further phospholipid compounds different from phosphatidylcholine and the anionic phospholipid compound, in particular one or more of the following phospholipid compounds: phosphatidylethanolamine, N-acylphosphatidylethanolamine and lysophosphatidylcholine. The total amount of phospholipid compounds different from phosphatidylcholine and the anionic phospholipid compound is typically in the range of 1.0 to 50.0% by weight, in particular in the range of 1.5 to 45.0% by weight, especially 2 in the range of.0 to 40.0% by weight, based on the total weight of the sunflower phospholipid composition. For example, the amount of lysophosphatidylcholine may be from traces up to 25.0% by weight, based on the total weight of the sunflower phospholipid composition. Likewise, the amount of phosphatidylethanolamine may be from traces up to 25.0% by weight, based on the total weight of the sunflower phospholipid composition and is frequently in the range of 1.0 to 20.0% by weight. Typically, the amount of N-acylphosphatidylethanolamine will not exceed 10.0% by weight and may be present from traces up to 10.0% by weight, based on the total weight of the sunflower phospholipid composition.

These mixtures of a sunflower phospholipid composition and at least one anionic stabilizer compound comprising at least one anionic phospholipid compound and especially the sunflower phospholipid compositions of the third aspect of the present invention can be obtained from a sunflower lecithin source by enrichment of the sunflower lecithin with regard to phosphatidylcholine, in particular by enrichment to a phosphatidylcholine level of not more than 80% by weight, in particular in the range of 45 to 75% by weight, based on the weight of the sunflower phospholipid composition. They may also be prepared by supplementing a conventional sunflower phospholipid composition containing phosphatidylcholine by conventional mixing techniques. In particular, these mixtures and especially the sunflower phospholipid compositions of the third aspect of the present invention can be prepared by a process which comprises dissolving a sunflower phospholipid composition containing phosphatidylcholine and at least one anionic stabilizer, in particular an anionic phospholipid compound in an organic solvent and evaporating the solvent. Typical solvents are organic solvent and mixtures of organic solvents, wherein the components, i.e. the sunflower phospholipid composition containing phosphatidylcholine and the anionic phospholipid compound are completely soluble in the required amounts. Suitable solvents include C₁-C₄ alkanols, such as methanol or ethanol, and mixtures thereof with water. Halogenated hydrocarbons such as dichloromethane and trichloromethane and mixtures thereof with the aforementioned solvents may also be used but are less preferred for toxicological and environmental reasons.

The sunflower phospholipid compositions as described herein, in particular the sunflower phospholipid compositions according to groups 1 and 2 of embodiments as well as the preferred sunflower phospholipid compositions and the sunflower phospholipid compositions of the second and the third aspect of the present invention may contain pharmaceutically acceptable antioxidants, in particular lipophilic antioxidants, e.g. tocopherols, such as alpha tocopherol, alpha tocopherol acetate, delta tocopherol, gamma tocopherol, vitamin E, ascorbyl palmitate, which may be synthetic or which may be of natural sources such as animal or preferably vegetable sources.

The sunflower phospholipid compositions as described herein, in particular the sunflower phospholipid compositions according to groups 1 and 2 of embodiments as well as the preferred sunflower phospholipid compositions and the sunflower phospholipid compositions of the second and the third aspect of the present invention will meet the pharmacopeial standards, in particular with regard to endotoxins and microbial content.

The sunflower phospholipid compositions as described herein, in particular the sunflower phospholipid compositions according to groups 1 and 2 of embodiments as well as the preferred sunflower phospholipid compositions and the sunflower phospholipid compositions of the second and the third aspect of the present invention are particularly suitable as emulsifiers in parenteral fat emulsions. Preformed mixtures of the third aspect of the invention may be used as such or combined with one or more further anionic stabilizer compounds.

They are particularly suitable as emulsifiers for aqueous oil-in-water emulsions for parenteral nutrition. They may, however, also be used as emulsifiers in parenteral fat emulsions of lipophilic drugs.

These aqueous oil-in-water emulsion for parenteral administration, in particular for parenteral nutrition, contain
a) an oil phase comprising a triglyceride composition suitable for parenteral administration, in particular for parenteral nutrition,
b) the sunflower phospholipid composition containing less than 0.1% by weight, in particular not more than 0.08% by weight or not more than 0.05% by weight and especially not more than 0.02% by weight of chlorogenic acids, based on the total weight of the sunflower phospholipid composition as defined herein in combination with at least one anionic stabilizer comprising at least one anionic phospholipid compound and optionally a further anionic stabilizer, in particular a combination of a sunflower phospholipid composition according to one of groups 1 or 2 of embodiments or a sunflower phospholipid composition according to the aspect 3 of the invention, and
c) water.

The kind of oil phase will depend in a known manner from the intended purpose of the aqueous oil-in-water emulsion. In case of an oil-in-water emulsion for parenteral nutrition the oil phase will essentially consist of a triglyceride compositions suitable for parenteral administration. In the context of the triglyceride compositions suitable for parenteral administration the term "consisting of" is understood that the oil phase contains to at least 95% of its weight, in particular at least 99% of its weight of the triglyceride composition.

Suitable triglyceride compositions for parenteral administrations, in particular for parenteral nutrition include oils and fats, in particular oils and fats from vegetable origin and oils of marine organisms, which include, but are not limited to soybean oil, safflower oil, sunflower oil, coconut oil, olive oil, medium chain triglycerides (MCT), long chain triglycerides (LCT), fish oil, krill oil, algae oil and mixtures of these oils, such as soybean oil/olive oil mixtures, soybean oil/safflower oil mixtures, soybean oil/MCT mixtures, inter-esterified mixtures of LCT and MCT, and soybean oil/MCT/olive oil/fish oil mixtures. The aforementioned oils mainly consist of triglycerides but may of course contain miner amounts of fatty acids. Typically the oils and fats consist of at least 99% by weight of triglycerides of fatty acids and optionally free fatty acids.

The term "fish oil" refers to "purified fish oil" and to "purified fish oil rich in omega 3 fatty acids", the latter according to the European Pharmacopoeia 6.0 comprising at least 9% (w/w) of the omega-3-fatty acid docosahexaenoic acid (DHA) and at least 13% (w/w) of the omega-3 fatty acid eisosapentaenoic acid (EPA) expressed as triglycerides.

For parenteral nutrition it is preferred that the triglycerides and free fatty acids contain a high amount of polyunsaturated fatty acids, including omega-3 fatty acids, in particular eicosapentaenoic acid (EPA) and docosahexaenoic acid (DHA), both of which are usually present in marine fish oils, and a-linolenic acid (ALA), commonly found in plant oils.

If the oil-in-water emulsion is intended for parenteral administration of a drug, the oil phase typically contains a drug, which is dissolved or dispersed in the oil phase. In particular, the drug is a lipophilic drug. Suitable drugs for parenteral administration may be from the groups of anaesthetics, analgetics, vasodilatators, immunosuppressants, anxiolytics, anti-emetics and antibiotics/antifungals and cytostatica. They include but are not limited to propofol, aprepitant, clevidipine butyrate, alprostadil, dexamethasone palmitate, diazepam, amphothericin B, etomidate, flurbiprofen axetil, prostaglandin E1 and cyclosporin, progesterone, lomustine, physostigmine salicylate and perilla ketone (1-(3-furanyl)-4-methyl-1-pentanone).

In the oil-in-water emulsion for parenteral administration the amount of oil phase is typically in the range of 5 to 35% by weight, in particular in the range of 8 to 30% by weight, more particularly in the range of 8 to 25% by weight, especially in the range of 8 to 17% by weight or in the range of 8 to 12% by weight or in the range of 15 to 22% by weight or in the range of 18 to 22% by weight, based on the total weight of the aqueous oil-in-water emulsion, of the oil phase.

The oil-in-water emulsion for parental administration also contains a sunflower phospholipid composition as defined herein, in particular a sunflower phospholipid composition according to one of the groups of embodiments 1 or 2 and especially a sunflower phospholipid composition according to one of the aspects 2 or 3 of the present invention.

The amount of the sunflower phospholipid composition is generally chosen such that the concentration of phosphatidylcholine in the oil-in-water emulsion is in the range of 0.1 to 10% by weight, in particular 0.2 to 5% by weight, especially 0.3 to 3% by weight, based on the total weight of the water-in-oil emulsion. In particular, the amount of the sunflower phospholipid composition is chosen such that the weight ratio of phosphatidylcholine and oil phase in the oil-in-water emulsion is in the range of 0.5 : 95 to 1 : 3, in particular 1 : 99 to 1 : 5 and especially 2 : 98 to 1 : 8.

As pointed out above, it is beneficial for the stability of the oil-in-water emulsion, if it contains at least one anionic stabilizer compound in addition to the phosphatidylcholine of the sunflower phospholipid compositions. According to the invention, the anionic stabilizer compound comprises or is at least one anionic phospholipid compound, in particular at least one anionic phospholipid compound, which is selected from the group consisting of phosphatidylglycerol, phosphatidylinositol, phosphatidylserine, phosphatidic acid, 1,3-bis(sn-3'-phosphatidyl)-sn-glycerol and combinations thereof. In emulsion, the anionic stabilizer compound may be present in its free acid form or in the form of a pharmaceutically acceptable salt thereof. In these emulsions, the anionic stabilizer compound is or comprises even more preferably phosphatidylglycerol, in particular phosphatidylglycerol obtained from sunflower, especially DMPG, or a pharmaceutically acceptable salt thereof, especially its sodium salt. In these emulsions, the weight ratio of phosphatidylcholine to the total amount of anionic stabiliser is generally in the range of 99.8 : 0.2 to 70 : 30, frequently in the range of 99.8 : 0.2 to 85.0 : 15.0, in particular in the range of 99.5 : 0.5 to 90.0 : 10.0, especially in the range of 99.0 : 1.0 to 90.0 : 10.0 and especially in the range of 98.5 : 1.5 to 91.0 : 9.0. The concentration of the anionic phospholipid in the emulsion is typically in the range of 0.005 to 0.2% by weight, in particular in the range of 0.01 to 0.15% by weight, based on the total weight of the emulsion. The total concentration of the anionic anionic stabilizer compounds in the emulsion is typically in the range of 0.005 to 0.25% by weight, in particular in the range of 0.01 to 0.2% by weight, based on the total weight of the emulsion.

As pointed out above, it is beneficial for the visual appearance, in particular for reducing or avoiding discoloration and the stability of the oil-in-water emulsion of the present invention, if it contains only small amounts or no chlorogenic acids. Therefore, the sunflower phospholipid composition contained in the emulsion has a content of chlorogenic acids of less than 0.1% by weight, in particular at most 0.08% by weight, especially at most 0.05% by weight, based on the total weight of the sunflower phospholipid composition. Typically, the total amount of chlorogenic acid in the emulsion does not exceed 0.001% by weight, in particular 0.0005% by weight and especially 0.0002% by weight, based on the total weight of the emulsion.

Apart from the aforementioned compounds the oil-in-water emulsion of the present invention may comprise at least one pharmaceutically acceptable antioxidant. An antioxidant useful in the emulsion of the disclosure may be any pharmaceutically acceptable compound having antioxidant activity, for example, the antioxidant may be selected form the group consisting of sodium metasulfite, sodium bisulfite, sodium sulfite, sodium thiosulfate, thioglycerol, thiosorbitol, thioglycolic acid, cysteine hydrochloride, n-acetyl- cysteine, citric acid, alpha-tocopherol, beta-tocopherol, gamma-tocopherol, delta-tocopherol, tocotrienols, soluble forms of vitamin E, butylated hydroxyanisole (BHA), butylated hydroxytoluene (BHT), t-butylhydroquinone (TBHQ), monothioglycerol, propyl gallate, histidine, enzymes such as superoxide dismutase, catalase, selenium glutathione peroxidase, phospholipid hydroperoxide and glutathione peroxidase, Coenzyme Q10, carotenoids, quinones, bioflavonoids, polyphenols, bilirubin, ascorbic acid, isoascorbic acid, uric acid, metal-binding proteins, ascorbic acid palmitate, and mixtures thereof.

The at least one antioxidant is in particular selected from the group consisting of alpha-tocopherol, beta-tocopherol, gamma-tocopherol, delta-tocopherol, tocotrienols, ascorbic acid, and mixtures of two or more thereof. Preferably, the antioxidant is alpha-tocopherol.

If present, the total amount of agents with antioxidant activity is preferably in the range of from 1 to 200 mg/L, preferably from 10 to 200 mg/L, more preferably from 40 to 150 mg/L, and even more preferably from 50 to 120 mg/L or from 75 to 100 mg/L.

The emulsion may comprise one or more pharmaceutically acceptable tonicity agents. Tonicity agents are used to confer tonicity to the emulsions. Suitable tonicity agents may be selected from the group consisting of sodium chloride, mannitol, lactose, dextrose, sorbitol and glycerol. Preferably, the tonicity agent is glycerol.

The amount of tonicity agents will depend on the desired osmolality and the molecular weight of the tonicity agent in a known manner. Usually, the total amount of tonicity agents is in the range of 0.1 to 10% by weight, more preferably from 1 to 5% by weight, more preferably from 1 to 4% by weight, more preferably 1 to 3.5% by weight, especially from 1.5 to 3.0% by weight, based on the total weight of the emulsion. In case the tonicity agent is glycerol the most preferred amount is 2.0 to 3.0% by weight, based on the total weight of the emulsion.

Preferably, the emulsion has an osmolality in the range of 180 to 300 mOsmol/L, in particular 190 to 280 mOsmol/L especially 200 to 250 mOsmol/L.

The aqueous oil-in-water emulsion of the present invention may have a pH value in the range of pH 4 to pH 10, as determined at 20°C and 1 bar. Preferably, the pH of the emulsion is in the range of from pH 6 to pH 9, such as pH 6.1 to about pH 8.9, or pH 6.2 to pH 8.8, or pH 6.3 to pH 8.7, or pH 6.4 to pH 8.6, or pH 6.5 to pH 8.5, or pH 6.6 to pH 8.4, or pH 6.7 to pH 8.3, or pH 6.8 to pH 8.2, or pH 6.9 to pH 8.1, or pH 7 to pH 8, or pH 7.1 to pH 7.9, or pH 7.2 to pH 7.8, or pH 7.3 to pH 7.7, or pH 7.4 to pH 7.6, e.g. about pH 7, about pH 7.5, and/or about pH 8.

The pH of the lipid emulsions may be adjusted by adding solutions of conventionally known acids or bases such as HCl and NaOH or, less preferred, through the use of buffers, such as phosphate buffers. Preferably, the pH of the emulsion according to the disclosure is adjusted using a solution of NaOH.

In the parenteral fat emulsions of the present invention the continuous phase is aqueous and disperse phase are formed by droplets of the oil phase, hereinafter also termed oil droplets. These oil droplets are stabilized within the aqueous phase by the sunflower phospholipid compositions as described herein, in particular by the phosphatidylcholine contained therein, and optionally further anionic stabilizers. The size of the oil droplets depends on the qualitative and quantitative composition of the emulsion and its preparation. The oil droplets of the emulsion herein preferably have a mean diameter of below 500 nm, e.g. in the range of 130 to 450 nm, preferably in the range of 150 to 400 nm, especially in the range of 180 to 350 nm. The mean diameters given here refer to the volume or mass moment mean, also termed as De Broucker mean, as determined by dynamic light scattering of the diluted aqueous emulsion at 20°C in accordance with ISO 13320:2009. The aqueous oil-in-water emulsion of the present invention will typically have PFAT5 value according to USP <729> of not more than 0.05%, which means that the volume weighted percentage of oil droplets/fat globules having a diameter of above 5 µm does not exceed 0.05% by weight.

Depending on the desired purpose, the parenteral fat emulsions according to the present invention may comprise one or more further additives present in the aqueous phase. For example, parenteral fat emulsions for parenteral nutrition may contain one or more additives, selected form amino acids, including essential amino acids, such as histidine, isoleucine, leucine, lysine, methionine, phenylalanine, threonine, tryptophan, and valine and mineral salts or pharmaceutically acceptable ammonium compounds such as carnitine or choline and pharmaceutically acceptable salts or derivatives thereof.

For example, parenteral fat emulsions for parenteral nutrition may further comprise L-carnitine or a salt or derivative thereof and/or choline or a salt or derivative thereof. Preferably, L-carnitine is provided in free form. Preferably, the parenteral fat emulsions according to the present disclosure comprise L-carnitine at a concentration of 500 to 1500 mg per litre, preferably at a concentration of 600 to 1000 mg per litre based on the total volume of the emulsion.

For example, parenteral fat emulsions for parenteral nutrition may further comprise choline. Preferably, choline is provided in form of cytidine 5'-diphosphocholine sodium, choline chloride or alpha-glyceryl phosphoryl choline (GPC). The GPC may be of any origin but is preferably obtained from a sunflower source. Preferably, parenteral fat emulsions may comprise choline at a concentration of 1 to 2 g per litre, preferably at a concentration of 1.1 to 1.7 g per litre based on the total volume of the emulsion.

For example, parenteral fat emulsions for parenteral nutrition may comprise both L- carnitine or a salt or derivative thereof and choline or a salt or derivative thereof.

The parenteral fat emulsions of the present invention can be prepared by analogy to generally known processes as described in the references cited in the background above, for example, in Hippalgaonkar et al, AAPS Pharm Sci Tech 2010, 11(4), 1526-1540) and the references cited therein.

Generally, the oil phase is emulsified in the aqueous phase in the presence of the phospholipid composition and the optional anionic stabilizer compounds. Emulsification is typically carried out in two steps comprising a first pre-emulsifying step, where a coarse emulsion is obtained, which then subjected to a homogenization step to reduce the droplet size of the oil droplets of the coarse emulsion to a to a droplet size suitable for parenteral administration. Hence, the process for preparing comprises the following steps:
i. Providing an aqueous phase and an oil phase;
ii. Emulsifying the oil phase in the aqueous phase
   - in the presence of sunflower phospholipid composition containing less than 0.1% by weight, in particular not more than 0.08% by weight or not more than 0.05% by weight and especially not more than 0.02% by weight of chlorogenic acids, based on the total weight of the sunflower phospholipid composition, as defined herein, and at least one anionic stabilizer comprising at least one anionic phospholipid compound as defined herein and optionally a further anionic stabilizer compound, or
   - in the presence of the sunflower phospholipid composition of the third aspect of the present invention;
iii. Homogenizing the emulsion obtained in step ii. to a droplet size suitable for parenteral administration.

Of course, the sunflower phospholipid composition used in step ii. has a content of chlorogenic acids of less than 0.1% by weight, in particular not more than 0.08% by weight, or not more than 0.05% by weight, especially not more than 0.02% by weight, based on the total weight of the sunflower phospholipid composition.

Typically water soluble ingredients are dissolved in the aqueous phase and oil-soluble ingredients, including drug substances, are dissolved in the triglyceride composition of the oil phase, respectively. For example, a choline source, an amino acid or a carnitine source, if present, will be dissolved in the aqueous phase. The sunflower phospholipid composition, can be dissolved in the oil phase or dispersed in the aqueous phase. Likewise, the anionic stabilizers can be dispersed in the oil phase or the aqueous phase. In preferred group of embodiments the sunflower phospholipid composition and the optional anionic stabilizer is dispersed in the aqueous phase prior to the emulsification step.

It is beneficial for the stability of the obtained emulsion and also for the reproducibility of the process if the combination is used as a preformed mixture as described above. The preformed mixture is then either dispersed in in the aqueous phase or dissolved in the oil phase. However, it is possible to disperse/dissolve the sunflower phospholipid composition and the anionic stabilizer separately in either the oil phase or the aqueous phase.

Before carrying out step ii., both phases may be adequately heated and optionally stirred to disperse or dissolve the ingredients. The temperature for heating will be typically in the range of 30 to 80°C.

In step ii. the oil-phase is then generally introduced to the aqueous phase and agitation, e.g. by using a high-shear mixer, to form a homogenously dispersed coarse or pre-emulsion. Pre-emulsions with a droplet size smaller than 20 µm generally produces unimodal and physically stable fine emulsions. Preferably, step ii. is carried out at elevated temperatures, e.g. in the range of 40 to 80°C. Preferably, the oil phase and the aqueous phase are preheated to the desired temperature of preferably in the range of 40 to 80°C and the heated oil phase and the heated aqueous phase are then mixed for emulsification.

The pre-emulsion is then homogenized, e.g. by using a microfluidizer or a high-pressure homogenizer, to further reduce the droplet size and form a fine emulsion having a particle size suitable for parenteral administration.

The required pressure, operating temperature, number of homogenization cycles, required for carrying out steps iii. of the process can be found by routine based on the examples disclosed herein. Likewise, other parameters such as type, composition and concentration of oil phase and of the aqueous phase may influence the mean droplet size during high-homogenization and the above parameters will be adjusted accordingly in a known manner. If pressure homogenization is used in step iii. the pressure may vary over a broad range. Generally, it will be in a range of 100 to 1300 bar, in particular in the range of 200 to 1200 bar, especially in the range of 300 to 110 bar, or 400 to 1000 bar.

Preferably, step iii. is carried out at elevated temperatures, e.g. in the range of 30 to 80°C.

If required, the pH of the homogenized parenteral fat emulsion obtained in step iii. may be adjusted to the desired value described above. However, it is also possible to adjust the pH before carrying out step ii. or between steps ii. and iii. It is also possible to adjust the pH between steps ii. and iii. and after step iii.. It was found beneficial, if the pH is adjusted before step ii. and/or before step iii. to a value of at least pH 6, in particular at least pH 7, e.g. to pH 7 to 9 to facilitate emulsification and homogenization, respectively. It should also be noted that the pH may be adjusted to pH which is slightly higher than the desired pH of the final emulsion, because the pH of the emulsion may decrease during emulsification, homogenization or sterilization.

The entire process (filtration/coarse and fine emulsion preparation) is usually carried out under nitrogen atmosphere whenever possible and especially in cases where the excipients and specific components of the lipid emulsion are sensitive to oxidation.

The homogenized parenteral fat emulsion obtained in step iii. will usually subjected to a sterilization step. Sterilization can be done by methods known in the art, e.g. by heat or by aseptic filtration.

Sterilization of the parenteral fat emulsions is preferably carried out by terminal heat sterilization, also termed autoclaving. Heat sterilization generally provides greater assurance of sterility of the final product. Moreover it was surprisingly found that autoclaving provides for an improved stability of the parenteral fat emulsion against phase separation or droplet growth. However, if the components of the emulsions are heat labile, sterile filtration can be used. Sterilization by filtration requires the emulsion droplet size to be below 200 nm.

Alternatively, aseptic processing may be employed. However, this process is relatively equipment and labour intensive and requires additional process validation data and justification during regulatory submissions.

In a very preferred group of embodiments, the process for preparing the parenteral fat emulsions according to the invention comprises the following steps:
a. Separately heating up the oil phase and the aqueous phase to a temperature of from about 40°C to about 80°C under agitation;
b. Dispersing the sunflower phospholipid composition with or without a stabiliser in the aqueous phase;
c. Preparing the pre-emulsion by transferring the oil phase to the aqueous phase under agitation;
d. Homogenizing the pre-emulsion at a temperature of from about 40°C to 80°C under pressure;
e. Optionally adding water to adjust the required volume and concentrations;
f. Adjusting the pH to a range of from about 4 to 10, in particular 6 to 9 or 7 to 9; and
g. Optionally sterilizing the lipid emulsion, by means of autoclaving and or sterile filtration.

Usually, at least steps a. through d. and optionally also steps e., f. and g. will be performed in the presence of an inert gas, such as N₂, for avoiding any oxidation reactions. A skilled person will readily appreciate that steps b., c., d., e. and g. are carried out in this order, and that step f. can be carried at any stage of the process but after step a. and before step g. Step a. may be carried out before or parallel to step b. but in any case before step c.. Step f. can be carried out between step c. and d. and/or after steps d. or e. or between steps b. and c. and/or after steps d. or e.

The parenteral fat emulsions of the present invention obtained in step iii., d., e., f. or g. are then packaged into suitable containers. Plastic containers which are permeable to oxygen and/or contain oil-soluble plasticizers and usually avoided.

For example, the parenteral fat emulsions of the present invention are packaged in a glass bottle with a suitable rubber stopper and aluminium closure or piggyback, meeting pharmaceutical requirements.

The parenteral fat emulsions of the present invention may also be packaged in one of the chambers of a multi-chamber container for parenteral administration of a nutritional formula. For example, the container may be in the form of a bag having multiple compartments or chambers. The container, such as a bag, includes at least two chambers, but may also contain three, four, or five chambers, and in one preferred embodiment, two or three chambers. Suitable containers, including soft bags, typically are sterile, non-pyrogenic, single-use, and/or ready-to-use. The multi-chamber containers are particularly useful for holding a pediatric and/or neonatal parenteral nutrition product and generally provide a parenteral fat emulsion as disclosed herein in the first chamber, an amino acid formulation in a second chamber, and a as carbohydrate formulation in a third chamber of the container.

The multi- chamber container, such as a three- chamber bag, may include vertical chambers. Suitable multi-chamber containers are disclosed in U.S. Patent Publication No. 2007/0092579. For example, the multi-chamber container may be configured as a bag that includes two or three adjacent chambers or compartments. If desired, frangible barriers or openable seals (e.g., peel seals or frangible seals) are used to separate the chambers of the multi- chamber container. Multi-chamber containers may also comprise three chambers for accommodating a parenteral fat emulsion, a carbohydrate formulation and an amino acid formulation, and further comprise at least one, in certain embodiments two or three smaller chambers which contain, for example, vitamin formulations and/or trace element formulations. In one specific embodiment, the multi- chamber container of the invention has a first chamber containing the parenteral fat emulsion according to the invention, a second chamber containing an amino acid formulation, a third chamber containing a carbohydrate formulation, a fourth chamber containing a vitamin formulation and a fifth chamber containing a trace element formulation.

The openable seals of said multi-chamber containers permit formulations to be separately stored and admixed/reconstituted just prior to administration thereby allowing storage in a single container of formulations which should not be stored as an admixture for an extended period of tune. Opening of the seals allows communication between the chambers and mixing of the contents of the respective chambers. The outside seals of the multi-chamber container are strong seals that do not open under the fluid pressure supplied to open the weaker peel seals or frangible seals between the chambers. In some embodiments, the openable seals of the multi-chamber container may be designed to allow for the admixing or reconstitution of only selected chambers of the multi-chamber container, for example, the admixing of the parenteral fat emulsion with the vitamin chamber and the amino acid chamber, if so desired.

The multi-chamber container may be provided with instructions explaining a desired order with which to open the peel seals, so that constituent fluids are mixed in a desired order. The unsealing strengths of the two or more peel seals may be varied to promote the opening of the seals in the desired order. For example, the unsealing strength of the peel seal to be opened first may be 1/3 to 1/2 of the unsealing strength required to open the peel seal to be opened second.

### Abbreviations:

- % b.w.: % by weight
- DMPG-Na: dimyristoyl phosphatidylglycerol, sodium salt
- DOPS-Na: Dioleoyl-glycero-3-phosphoserine, sodium salt
- LPC: lysophosphatidylcholine
- m: month
- n.d.: not determined
- n.l.t.: not less than
- n.m.t: not more than
- PC: Phosphatidylcholine
- Pdl: Polydispersity index
- PE: Phosphatidylethanolamine
- PG: Phosphatidylglycerol
- RT: room temperature, i.e. 22°C
- w: weeks

### Analytics and assessment of quality/stability:

pH values were determined using a calibrated portable pH meter equipped with a glass pH electrode (Portamess^{®} 911 pH, Knick GmbH, Berlin, Germany).

The mean particle size of the emulsions was determined by dynamic light scattering technique on a ZetasizerNano-ZS90 (Malvern Instruments, Malvern, UK). Emulsions were diluted with water for injection to an appropriate concentration for measurement. Measurement was performed at 20°C. The intensity-weighted mean droplet diameter is reported.

Acid values were determined according to Ph.Eur. method. All values reported are given as mg KOH/g.

Compositions of the sunflower phospholipid compositions were assessed by standard HPLC protocols as described e.g. in van Hoogevest, P. and Wendel, A., "The use of natural and synthetic phospholipids as pharmaceutical excipients." European journal of lipid science and technology 116(9) (2014): 1088-1107.

For visual inspection, the emulsions were transferred to 50 mL glass vials and inspected visually for free fat droplets under a suitable light source.

For stability testing, the vials were stored at ca. 20-25°C in the dark and analyzed at defined time points. For accelerated storage condition testing, the vials were stored in a heating oven (Memmert, Schwabach, Germany) at 45°C under exclusion of light and analyzed after six weeks and three months.

### Properties/Qualities of the chemicals used

Phospholipid composition P1: Purified fraction of a sunflower lecithin. The purified fraction contained n.l.t. 96% b.w. PC, n.m.t. 2% b.w. LPC, <0.1% b.w. PE, <0.1% b.w. N-acylphosphatidylethanolamine, <0.05% b.w. free fatty acids and n.m.t. 1.7% b.w. triglycerides.

The acid value was 0.1-0.3, the iodine value was 96-108, and it exhibited a fatty acid spectrum typical for sunflower phospholipids (ca. 9% palmitic acid, ca. 3% b.w. stearic acid, ca. 16% b.w. oleic acid, and ca. 70% b.w. linoleic acid). Chlorogenic acid content was below 0.03% b.w..

Phospholipid composition P2: Purified fraction of a sunflower lecithin. The purified fraction contained 64% b.w. PC, 1.9% b.w. LPC, 6.9% b.w. PE and 0.3% b.w. triglycerides. The acid value was 18, and it exhibited a fatty acid spectrum typical for sunflower phospholipids (ca. 9% b.w. palmitic acid, ca. 3% b.w. stearic acid, ca. 16% b.w. oleic acid, and ca. 70% b.w. linoleic acid). Chlorogenic acid content was 1.3% b.w..

Phospholipid composition P3: Purified fraction of a sunflower lecithin. The purified fraction contained 71% b.w. PC, 2.0% b.w. LPC, 0.9% b.w. PG and 7.7% b.w. PE. The acid value was 11, and it exhibited a fatty acid spectrum typical for sunflower phospholipids (ca. 9% b.w. palmitic acid, ca. 3% b.w. stearic acid, ca. 16% b.w. oleic acid, and ca. 70% b.w. linoleic acid). Chlorogenic acid content was 0.06% b.w..

Phospholipid composition P3 was prepared as follows: Deoiled sunflower lecithin having a chlorogenic acid content of 0.64% b.w. was extracted five times with acetone having a water content of approx. 20% b.w.. The residue was dried under vacuum. The dried residue was then extracted with ethanol (96%) and filtered. The extraction was repeated twice. The filtrates were combined and evaporated to dryness under vacuum, yielding 13% (with respect to the deoiled lecithin) of phospholipid composition P3.

Phospholipid composition P4: Purified fraction of a sunflower lecithin having a similar overall composition as phospholipid composition P1 but having a chlorogenic acid content of 1.1% b.w..

Phospholipid composition P5: Mixture of phospholipid composition P1 and DMPG-Na in a weight ratio 20 : 1 (ratio of PC : DMPG-Na = 19.6 : 1)

A phospholipid composition P5 was prepared by dissolving the phospholipid composition P1 (50 g) and DMPG-Na (2.5 g) in a mixture of chloroform/methanol/water at 50°C. The solvents were evaporated under vacuum and the resulting waxy solid was further dried at 30°C under vacuum.

Phospholipid composition P6: Mixture of phospholipid composition P1 and DMPG-Na in a weight ratio 15.4 : 1 (ratio of PC : DMPG-Na = 15.1 : 1)

Phospholipid composition P6 was prepared by dissolving the phospholipid composition P1 (20 g) and DMPG-Na (1.3 g) in a mixture of chloroform/methanol/water at 50°C. The solvents were evaporated under vacuum and the resulting waxy solid was further dried at 25°C under vacuum.

Phospholipid composition P7: Mixture of phospholipid composition P1 and DMPG-Na in a weight ratio 60.6 : 1 (ratio of PC : DMPG-Na = 59.4 : 1)

Phospholipid composition P7 was prepared by dissolving the phospholipid composition P1 (20 g) and DMPG-Na (0.33 g) in a mixture of chloroform/methanol/water at 50°C. The solvents were evaporated under vacuum and the resulting waxy solid was further dried at 25°C under vacuum.

Phospholipid composition P8: Mixture of phospholipid composition P1 and DMPG-Na in a weight ratio 20 : 1 (ratio of PC : DOPS-Na = 19.6 : 1)

A phospholipid composition P8 was prepared by dissolving the phospholipid composition P1 (20 g) and DOPS-Na (1.0 g) in a mixture of chloroform/methanol/water at 50°C. The solvents were evaporated under vacuum and the resulting waxy solid was further dried at 25°C under vacuum.

DMPG-Na (Dimyristoyl phosphatidylglycerol, sodium salt) was used as a commercial product (LIPOID GmbH, Ludwigshafen, Germany). It had a chromatographic purity of ≥98%. The amount of myristic acid was ≥98.0% b.w. based on the total amount of fatty acid in DMPG-Na.

DOPS-Na (Dioleoyl-glycero-3-phosphoserine, sodium salt) was used as a commercial product (LIPOID GmbH, Ludwigshafen, Germany). It had a chromatographic purity of ≥90%. The purity of oleic acid was ≥98.0% based on the total amount of fatty acid in DOPS-Na.

Glycerol, sodium hydroxide, soybean oil and MCT oil (medium-chain triglycerides) qualities used met Ph.Eur. requirements. Lysine dihydrochloride (≥98%) and glycin (≥99%) were from Sigma-Aldrich.

### Experimental Conditions/Equipment

For all experiments, water for injection (Ph.Eur.) with a maximum conductivity of 5.0 µS/cm was used.

In the following experiments, an Ultra-Turrax T 50 (IKA Labortechnik, Staufen, Germany) was used for high shear mixing.

High pressure homogenization was carried out with a Microfluizider 110 T (Microfluidics^{™}, USA).

The parenteral fat emulsions prepared in the following examples in glass vials and autoclaved. Filling was carried out under nitrogen in glass vials (20 mL) of hydrolytic class 1. The glass vials were closed with bromobutyl rubber stoppers, crimped, and autoclaved in a steam autoclave (Systec GmbH, Linden, Germany) equipped with a fast cooling function. Hold time was 15 min at 121°C.

### Preparation Examples:

In the following preparation examples glycerol was used as a tonicity agent in an amount of 2.25% by weight. In each example, the amount of water was chosen such that the batch size was 500 g.

### Example 1 (not according to the invention)

Glycerol and water for injection were mixed. Phospholipid composition P1 (6.0 g) was added and the mixture was stirred at 50°C, until the phospholipid was completely dispersed. Purified soybean oil (100 g) was heated separately to 60°C. The aqueous phase was mixed with a high-shear mixer at 10.000 rpm. Mixing was continued while the heated oil phase was added slowly. pH was adjusted to 8.5 by adding a sodium hydroxide solution in water (0.5M). The resulting pre-emulsion was then homogenized using a high-pressure homogenizer in five passes at a pressure of 800 bar. Temperature was kept at 50°C to 70°C during homogenization. Prior to the 5^{th} pass, pH was again adjusted to 8.5. After the homogenization, the emulsion was cooled below 30°C, filled in glass vials and autoclaved.

### Analytical data:

Homogenous, white liquid
pH value: 7.4
Average particle size (Pdl): 229 nm (0.046)
Stability: Free fat visible after 3 months storage at RT

### Example 2

Glycerol and water for injection were mixed. Phospholipid composition P1 (6.0 g) and DMPG-Na (0.30 g) were added and the mixture was stirred at 50°C, until the phospholipids were completely dispersed. Purified soybean oil (100 g) was heated separately to 60°C. The aqueous phase was mixed with a high-shear mixer at 10.000 rpm. Mixing was continued while the heated oil phase was added slowly. pH was adjusted to 7.5 by adding a sodium hydroxide solution in water (0.5M). The resulting pre-emulsion was then homogenized using a high-pressure homogenizer in five passes at a pressure of 800 bar. Temperature was kept at 50°C to 70°C during homogenization. Prior to the 5^{th} pass, pH was again adjusted to 7.5. After the homogenization, the emulsion was cooled below 30°C, filled in glass vials and autoclaved.

### Analytical data:

Homogenous, white liquid
pH value: 6.8
Average particle size (Pdl): 220 nm (0.099)

### Stability: 3 m at 45°C:

Homogenous, white liquid
pH value: 6.5
Average particle size: 234 nm

### 6 m at room temperature:

Homogenous, white liquid
pH value: 7.7
Average particle size: 234 nm

### 18 m at room temperature:

Homogenous, white liquid
pH value: 7.6
Average particle size: 238 nm

### Example 3

Glycerol and water for injection were mixed. The phospholipid composition P5 (6.0 g) was added and the mixture was stirred at 50°C, until the phospholipids were completely dispersed. Purified soybean oil (100 g) was heated separately to 60°C. The aqueous phase was mixed with a high-shear mixer at 10.000 rpm. Mixing was continued while the heated oil phase was added slowly. pH was adjusted to 8.3 by adding a sodium hydroxide solution in water (0.5M). The resulting pre-emulsion was then homogenized using a high-pressure homogenizer in five passes at a pressure of 800 bar. Temperature was kept at 50°C to 70°C during homogenization. Prior to the 5^{th} pass, pH was again adjusted to 8.5. After the homogenization, the emulsion was cooled below 30°C, filled in glass vials and autoclaved.

### Analytical data:

Homogenous, white liquid
pH value: 7.7
Average particle size (Pdl): 245 nm (0.070)

### Stability: 6 w at 45°C:

Homogenous, white liquid
pH value: 7.9
Average particle size: 239 nm

### 3 m at 45°C:

Homogenous, white liquid
pH value: 7.9
Average particle size: 241 nm

### 12 m at room temperature:

Homogenous, white liquid
pH value: 7.9
Average particle size: 241 nm

### Example 4

Example 4 was carried out according to the protocol of example 1 using phospholipid composition P3 (6.0 g) instead of phospholipid composition P1 (6.0 g).

### Analytical data:

Homogenous, white liquid
pH value: 7.7
Average particle size (Pdl): 242 nm (0.095)

### Stability: 3 m at 45°C:

Homogenous, white liquid
pH value: 7.6
Average particle size: 250 nm

### 12 m at room temperature:

Homogenous, white liquid
pH value: 7.6
Average particle size: 260 nm

### Example 5 (not according to the invention)

Example 5 was carried out according to the protocol of example 1 using phospholipid composition P2 (6.0 g) instead of phospholipid composition P1 (6.0 g). After first pH adjustment, the pre-emulsion turned green. After autoclaving, the green color had faded and the emulsion had an off-white color.

### Analytical data:

Homogenous, off-white liquid
pH value: 7.6
Average particle size (Pdl): 253 nm (0.094)

### Stability: 6 w at 45°C:

Off-white liquid with small oil droplets on the surface
pH value: 7.0
Average particle size: 239 nm

### 3 m at 45°C:

Off-white liquid with free fat on the surface
pH value: 6.9
Average particle size: n.d.

### 12 m at room temperature:

Off-white liquid with small oil droplets on the surface
pH value: 7.2
Average particle size: 237 nm

### Example 6 (not according to the invention)

Example 6 was carried out according to the protocol of example 1 using a mixture of purified soybean oil (50 g) and purified medium-chain triglycerides (50 g) instead of purified soybean oil (100 g).

### Analytical data:

Homogenous, white liquid
pH value: 7.5
Average particle size (Pdl): 270 nm (0.023)

### Stability: 3 m at 45°C:

White liquid with small oil droplets on the surface
pH value: 6.9
Average particle size: 259 nm

### 12 m at room temperature:

White liquid with small oil droplets on the surface
pH value: 7.9
Average particle size: 276 nm

### Example 7

Glycerol and water for injection were mixed. Phospholipid composition P1 (6.0 g) and DMPG-Na (0.30 g) were added and the mixture was stirred at 50°C, until the phospholipids were completely dispersed. A mixture of purified soybean oil (50 g) and purified medium-chain triglycerides (50 g) was heated separately to 60°C. The aqueous phase was mixed with a high-shear mixer at 10.000 rpm. Mixing was continued while the heated oil phase was added slowly. pH was adjusted to 8.5 by adding a sodium hydroxide solution in water (0.5M). The resulting pre-emulsion was then homogenized using a high-pressure homogenizer in five passes at a pressure of 800 bar. Temperature was kept at 50°C to 70°C during homogenization. Prior to the 5^{th} pass, pH was again adjusted to 8.5. After the homogenization, the emulsion was cooled below 30°C, filled in glass vials and autoclaved.

### Analytical data:

Homogenous, white liquid
pH value: 7.8
Average particle size (Pdl): 227 nm (0.065)

### Example 8

Example 8 was carried out according to the protocol of example 3 using a mixture of purified soybean oil (50 g) and purified medium-chain triglycerides (50 g) instead of purified soybean oil (100 g). As in example 3 a homogeneous mixture of phospholipid composition P1 and DMPG-Na in a weight ratio of 20 : 1 was used (6 g of P5 instead of P1).

### Analytical data:

Homogenous, white liquid
pH value: 7.5
Average particle size (Pdl): 232 nm (0.026)

### Stability: 3 m at 45°C:

Homogenous, white liquid
pH value: 7.2
Average particle size: 212 nm

### Example 9

Example 9 was carried out according to the protocol of example 1 using a mixture of purified soybean oil (50 g) and purified medium-chain triglycerides (50 g) instead of purified soybean oil (100 g) and using phospholipid composition P3 instead of the phospholipid composition P1.

### Analytical data:

Homogenous, white liquid
pH value: 7.8
Average particle size (Pdl): 240 nm (0.002)

### Stability: 3 m at 45°C:

Homogenous, white liquid
pH value: 7.1
Average particle size: 224 nm

### Example 10 (not according to the invention)

Glycerol and water for injection were mixed. Phospholipid composition P2 (6.0 g) was added and the mixture was stirred at 50°C, until the phospholipids were completely dispersed. A mixture of purified soybean oil (50 g) and purified medium-chain triglycerides (50 g) was heated separately to 60°C. The aqueous phase was mixed with a high-shear mixer at 10.000 rpm. Mixing was continued while the heated oil phase was added slowly. pH was adjusted to 8.5 by adding a sodium hydroxide solution in water (0.5M). After pH adjustment, the pre-emulsion turned green. The pre-emulsion was then homogenized using a high-pressure homogenizer in five passes at a pressure of 800 bar. Temperature was kept at 50°C to 70°C during homogenization. Prior to the 5^{th} pass, pH was again adjusted to 8.5. After the homogenization, the emulsion was cooled below 30°C, filled in glass vials and autoclaved. After autoclaving, the green color had faded and the emulsion had an off-white to greyish color.

### Analytical data:

Homogenous, off-white liquid
pH value: 7.6
Average particle size (Pdl): 243 nm (0.005)

### Stability: 3 m at 45°C:

Homogenous, greyish liquid
pH value: 7.0
Average particle size: 230 nm

### 12 m at room temperature:

Greyish liquid with small oil droplets on the surface
pH value: 7.7
Average particle size: 265 nm

### Example 11

Glycerol and water for injection were mixed. Phospholipid composition P1 (6.0 g) and DMPG-Na (0.30 g) were added and the mixture was stirred at 50°C, until the phospholipids were completely dispersed. Purified soybean oil (100 g) was heated separately to 60°C. Glycine (0.05 g) and lysine dihydrochloride (0.05 g) were added to the water phase and stirring was continued, until the amino acids were dissolved (ca. 2 min). The aqueous phase was mixed with a high-shear mixer at 10.000 rpm. Mixing was continued while the heated oil phase was added slowly. pH was adjusted to 7.5 by adding a sodium hydroxide solution in water (0.5M). The resulting pre-emulsion was then homogenized using a high-pressure homogenizer in five passes at a pressure of 800 bar. Temperature was kept at 50°C to 70°C during homogenization. Prior to the 5^{th} pass, pH was adjusted to 8.5. After the homogenization, the emulsion was cooled below 30°C, filled in glass vials and autoclaved.

### Analytical data:

Homogenous, white liquid
pH value: 8.6
Average particle size (Pdl): 245 nm (0.100)

### Stability: 3 m at 45°C:

Homogenous, white liquid
pH value: 7.9
Average particle size: 240 nm

### 12 m at room temperature:

Homogenous, white liquid
pH value: 8.4
Average particle size: 262 nm

### Example 12 (not according to the invention)

Glycerol and water for injection were mixed. Phospholipid composition P4 (6.0 g) and DMPG-Na (0.30 g) were added and the mixture was stirred at 50°C, until the phospholipids were completely dispersed. Purified soybean oil (100 g) was heated separately to 60°C. Glycine (0.05 g) and lysine dihydrochloride (0.05 g) were added to the water phase and stirring was continued, until the amino acids were dissolved (ca. 2 min). The aqueous phase was mixed with a high-shear mixer at 10.000 rpm. Mixing was continued while the heated oil phase was added slowly. pH was adjusted to 7.7 by adding a sodium hydroxide solution in water (0.5M). After pH adjustment, the pre-emulsion turned green. The resulting pre-emulsion was then homogenized using a high-pressure homogenizer in five passes at a pressure of 800 bar. Temperature was kept at 50°C to 70°C during homogenization. Prior to the 5^{th} pass, pH was adjusted to 8.5. After the homogenization, the emulsion was cooled below 30°C, filled in glass vials and autoclaved. After autoclaving, the green color had faded and the emulsion had a greyish color.

### Analytical data:

Homogenous, greyish liquid
pH value: 8.6
Average particle size (Pdl): 240 nm (0.033)

### Stability: 3 m at 45°C:

Homogenous, greyish liquid
pH value: 7.7
Average particle size: 234 nm

### 12 m at room temperature:

Greyish liquid with small oil droplets on the surface
pH value: 8.2
Average particle size: 260 nm

### Example 13

Example 13 was carried out according to the protocol of example 11 using a mixture of purified soybean oil (50 g) and purified medium-chain triglycerides (50 g) instead of purified soybean oil (100 g).

### Analytical data:

Homogenous, white liquid
pH value: 8.3
Average particle size (Pdl): 226 nm (0.102)

### Stability: 3 m at 45°C:

Homogenous, white liquid
pH value: 8.1
Average particle size: 224 nm

### Example 14 (not according to the invention)

Example 14 was carried out according to the protocol of example 12 using a mixture of purified soybean oil (50 g) and purified medium-chain triglycerides (50 g) instead of purified soybean oil (100 g). First pH adjustment was pH 7.8 instead of pH 7.7. After first pH adjustment, the pre-emulsion turned green. After autoclaving, the green color had faded and the emulsion had a greyish appearance.

### Analytical data:

Homogenous, greyish liquid
pH value: 8.3
Average particle size (Pdl): 237 nm (0.014)

### Stability: 3 m at 45°C:

Homogenous, greyish liquid
pH value: 7.4
Average particle size: 227 nm

### 12 m at room temperature:

Greyish liquid with small oil droplets on the surface
pH value: 8.1
Average particle size: 224 nm

### Example 15

Example 15 was carried out according to the protocol of example 2 using 0.4 g of DMPG-Na (0.08% b.w. based on the emulsion) instead of 0.3 g of DMPG-Na.

### Analytical data:

Homogenous, white liquid
pH value: 7.2
Average particle size (Pdl): 229 nm (0.105)

### Stability: 3 m at 45°C:

Homogenous, white liquid
pH value: 6.8
Average particle size: 244 nm

### 18 m at room temperature:

Homogenous, white liquid
pH value: 7.8
Average particle size: 246 nm

### Example 16

Example 16 was carried out according to the protocol of example 2 using 0.1 g of DMPG-Na (0.02% b.w. based on the emulsion) instead of 0.3 g of DMPG-Na.

### Analytical data:

Homogenous, white liquid
pH value: 6.9
Average particle size (Pdl): 221 nm (0.043)

### Stability: 3 m at 45°C:

Homogenous, white liquid
pH value: 6.8
Average particle size: 239 nm

### 18 m at room temperature:

Homogenous, white liquid
pH value: 7.3
Average particle size: 244 nm

### Example 17

Example 17 was carried out according to the protocol of example 7 using 0.4 g of DMPG-Na (0.08% b.w. based on the emulsion) instead of 0.3 g of DMPG-Na.

### Analytical data:

Homogenous, white liquid
pH value: 7.7
Average particle size (Pdl): 200 nm (0.032)

### Stability: 3 m at 45°C:

Homogenous, white liquid
pH value: 7.6
Average particle size: 218 nm

### 3 m at room temperature:

Homogenous, white liquid
pH value: 7.9
Average particle size: 209 nm

### Example 18

Example 18 was carried out according to the protocol of example 7 using 0.1 g of DMPG-Na (0.02% b.w. based on the emulsion) instead of 0.3 g of DMPG-Na.

### Analytical data:

Homogenous, white liquid
pH value: 7.7
Average particle size (Pdl): 209 nm (0.035)

### Stability: 3 m at 45°C:

Homogenous, white liquid
pH value: 7.2
Average particle size: 218 nm

### 3 m at room temperature:

Homogenous, white liquid
pH value: 7.2
Average particle size: 225 nm

### Example 19

Example 19 was carried out according to the protocol of example 3 using 6.0 g of phospholipid composition P6 instead of phospholipid composition P5.

### Analytical data:

Homogenous, white liquid
pH value: 7.8
Average particle size (Pdl): 232 nm (0.042)

### Stability: 3 m at 45°C:

Homogenous, white liquid
pH value: 7.7
Average particle size: 246 nm

### 3 m at room temperature:

Homogenous, white liquid
pH value: 8.2
Average particle size: 247 nm

### Example 20

Example 20 was carried out according to the protocol of example 3 using 6.0 g of phospholipid composition P7 instead of phospholipid composition P5.

### Analytical data:

Homogenous, white liquid
pH value: 7.7
Average particle size (Pdl): 222 nm (0.096)

### Stability: 3 m at room temperature:

Homogenous, white liquid
pH value: 8.0
Average particle size: 245 nm

### Example 21

Example 21 was carried out according to the protocol of example 8 using 6.0 g of phospholipid composition P6 instead of phospholipid composition P5.

### Analytical data:

Homogenous, white liquid
pH value: 7.6
Average particle size (Pdl): 210 nm (0.013)

### Stability: 3 m at room temperature:

Homogenous, white liquid
pH value: 8.1
Average particle size: 221 nm

### Example 22

Example 22 was carried out according to the protocol of example 8 using 6.0 g of phospholipid composition P7 instead of phospholipid composition P5.

### Analytical data:

Homogenous, white liquid
pH value: 7.8
Average particle size (Pdl): 206 nm (0.033)

### Stability: 3 m at room temperature:

Homogenous, white liquid
pH value: 8.2
Average particle size: 219 nm

### Example 24

Example 24 was carried out according to the protocol of example 3 using 6.0 g of phospholipid composition P8 instead of phospholipid composition P5.

### Analytical data:

Homogenous, white liquid
pH value: 8.2
Average particle size (Pdl): 212 nm (0.061)

### Stability: 3 m at 45°C:

Homogenous, white liquid
pH value: 7.7
Average particle size: 245 nm

### 6 m at room temperature:

Homogenous, white liquid
pH value: 8.0
Average particle size: 245 nm

### Example 25

Example 25 was carried out according to the protocol of example 7 using 6.0 g of phospholipid composition P1 and 0.3 g of DOPS-NA instead of DMPG-Na.

### Analytical data:

Homogenous, white liquid
pH value: 8.2
Average particle size (Pdl): 216 nm (0.071)

### Stability: 3 m at 45°C:

Homogenous, white liquid
pH value: 7.3
Average particle size: 221 nm

### Example 26

Example 26 was carried out according to the protocol of example 8 using 6.0 g of phospholipid composition P8 instead of phospholipid composition P5.

### Analytical data:

Homogenous, white liquid
pH value: 8.0
Average particle size (Pdl): 203 nm (0.013)

### Stability: 3 m at 45°C:

Homogenous, white liquid
pH value: 7.3
Average particle size: 226 nm

### 3 m at room temperature:

Homogenous, white liquid
pH value: 7.8
Average particle size: 228 nm

### Example 27

Example 27 was carried out according to the protocol of example 2 using additionally 0.15 g of sodium oleate (0.03% b.w. based on the emulsion). The sodium oleate was added together with phospholipid composition P1 (6.0 g) and DMPG-Na (0.3 g) to the water/glycerine mixture.

### Analytical data:

Homogenous, white liquid
pH value: 8.6
Average particle size (Pdl): 229 nm (0.102)

### Stability: 6 w at 45°C:

Homogenous, white liquid
pH value: 8.0
Average particle size: 240 nm

### Example 28

Example 28 was carried out according to the protocol of example 7 using additionally 0.15 g of sodium oleate (0.03% b.w. based on the emulsion). The sodium oleate was added together with phospholipid composition P1 (6.0 g) and DMPG-Na (0.3 g) to the water/glycerine mixture.

### Analytical data:

Homogenous, white liquid
pH value: 8.5
Average particle size (Pdl): 202 nm (0.064)

### Stability: 6 w at 45°C:

Homogenous, white liquid
pH value: 7.7
Average particle size: 208 nm

### Example 29

Example 29 was carried out according to the protocol of example 4 using additionally 0.15 g of sodium oleate (0.03% b.w. based on the emulsion). The sodium oleate was added together with phospholipid composition P3 (6.0 g) to the water/glycerine mixture.

### Analytical data:

Homogenous, white liquid
pH value: 8.3
Average particle size (Pdl): 236 nm (0.081)

### Stability: 3 m at 45°C:

Homogenous, white liquid
pH value: 7.7
Average particle size: 230 nm

### 12 m at room temperature:

Homogenous, white liquid
pH value: 7.9
Average particle size: 235 nm

Example 30 was carried out according to the protocol of example 9 using additionally 0.15 g of sodium oleate (0.03% b.w. based on the emulsion). The sodium oleate was added together with phospholipid composition P3 (6.0 g) to the water/glycerine mixture.

### Analytical data:

Homogenous, white liquid
pH value: 8.3
Average particle size (Pdl): 215 nm (0.056)

### Stability: 6 w at 45°C:

Homogenous, white liquid
pH value: 7.6
Average particle size: 234 nm

## Claims

1. A sunflower phospholipid composition containing phosphatidylcholine in an amount of at least 45% by weight, based on the total weight of the phospholipid composition, and containing less than 0.1% by weight of chlorogenic acids, based on the total weight of the sunflower phospholipid composition, in combination with at least one anionic stabilizer compound comprising at least one anionic phospholipid compound as an emulsifier in an aqueous oil-in-water emulsion for use in the parenteral administration.

2. The composition of claim 1, where the sunflower phospholipid composition contains at least one anionic phospholipid compound in addition to the phosphatidylcholine.

3. The composition of any one of claims 1 or 2, where the anionic phospholipid compound is selected from the group consisting of phosphatidylglycerol, phosphatidylinositol, PEGylated phosphatidylethanolamine, phosphatidylserine, phosphatidic acid, 1,3-bis(sn-3'-phosphatidyl)-sn-glycerol and combinations thereof, and where the anionic phospholipid compound is preferably phosphatidylglycerol, in particular 1,2-dimyristoyl-sn-glycero-3-phosphorylglycerol.

4. The composition of any one of claims 1 to 3, where the weight ratio of the anionic phospholipid compound and sunflower phospholipid composition is in the range of 0.2 : 99.8 to 20 : 80.

5. The composition of any one of the preceding claims, where the sunflower phospholipid composition contains phosphatidylethanolamine.

6. The composition of any one of the preceding claims, where the sunflower phospholipid composition is used in such an amount that the concentration of phosphatidylcholine in the oil-in-water emulsion is in the range of 0.1 to 20% by weight, based on the total weight of the water-in-oil emulsion.

7. The composition of any one of the preceding claims, where the oil-in-water emulsion for parenteral administration is an oil-in-water emulsion for parenteral nutrition.

8. A combination of a sunflower phospholipid composition containing phosphatidylcholine in an amount of at least 45% by weight, based on the total weight of the sunflower phospholipid composition and having a content of chlorogenic acids of less than 0.1% by weight, based on the total weight of the sunflower phospholipid composition, with at least one anionic stabilizer compound comprising at least one anionic phospholipid compound.

9. The combination of claim 8, which is a sunflower phospholipid composition containing phosphatidylcholine in an amount of at least 45% by weight, based on the total weight of the sunflower phospholipid composition, and at least one anionic stabilizer compound comprising at least one anionic phospholipid compound and having a content of chlorogenic acids of less than 0.1% by weight, based on the total weight of the sunflower phospholipid composition, where the composition is a mixture of the sunflower phospholipid composition and the anionic stabilizer.

10. The combination of claim 8 or claim 9, which has at least one of the following features 10.a to 10.b:
10.a the anionic phospholipid compound is selected from the group consisting of phosphatidylglycerol, phosphatidylinositol, PEGylated phosphatidylethanolamine, phosphatidylserine, phosphatidic acid, 1,3-bis(sn-3'-phosphatidyl)-sn-glycerol and combinations thereof;
10.b the weight ratio of the anionic phospholipid compound and phosphatidylcholine is in the range of 0.2 : 99.8 to 30 : 70.

11. An aqueous oil-in-water emulsion for parenteral administration, in particular for parenteral nutrition which contains
a) an oil phase comprising a triglyceride composition suitable for parenteral administration, in particular for parenteral nutrition,
b) a combination of the sunflower phospholipid composition containing less than 0.1% by weight of chlorogenic acids, based on the total weight of the sunflower phospholipid composition, with at least one anionic stabilizer compound, where the combination is as defined in any one of claims 8 to 10,
and
c) water.

12. The aqueous oil-in-water emulsion of claim 11, which contains
a) 5 to 35 by weight%, based on the total weight of the aqueous oil-in-water emulsion, of the oil phase,
b) the combination of the sunflower phospholipid composition containing less than 0.1% by weight of chlorogenic acids, based on the total weight of the sunflower phospholipid composition, with at least one anionic phospholipid compound, as defined in any one of claims 1 to 7,
where the amount of the sunflower composition is such that the concentration of phosphatidylcholine in the oil-in-water emulsion is in the range of 0.1 to 20% by weight, based on the total weight of the oil-in-water emulsion, and
c) water.

13. The aqueous oil-in-water emulsion of any one of claims 11 or 12, which has at least one of the features 13.a or 13.b:
13.a it contains less than 100 ppm of chlorogenic acids;
13.b it has a pH value in the range of pH 4 to pH 10, as determined at 20°C and 1 bar.

14. The aqueous oil-in-water emulsion of any one of claims 11 or 13, wherein the oil phase essentially consists of a triglyceride composition suitable for parenteral administration.

15. The aqueous oil-in-water emulsion of any one of claims 11 or 14, wherein the anionic phospholipid compound is selected from the group consisting of phosphatidylglycerol, phosphatidylinositol, PEGylated phosphatidylethanolamine, phosphatidylserine, phosphatidic acid, 1,3-bis(sn-3'-phosphatidyl)-sn-glycerol and combinations thereof.

16. The aqueous oil-in-water emulsion of any one of claims 11 or 15, wherein the weight ratio of the anionic phospholipid compound and phosphatidylcholine is in the range of 0.2 : 99.8 to 15 : 85.

17. A process for preparing an aqueous oil-in-water emulsion for parenteral administration as claimed in any one of claims 11 to 16 comprising
i. Providing an aqueous phase and an oil phase;
ii. Emulsifying the oil phase in the aqueous phase
- in the presence of the combination of the sunflower phospholipid composition containing less than 0.1% by weight of chlorogenic acids, based on the total weight of the sunflower phospholipid composition, with at least one anionic stabilizer compound comprising an anionic phospholipid compound, as defined in any one of claims 1 to 7;
iii. Homogenizing the emulsion obtained in step to a droplet size suitable for parenteral administration.

18. The process of claim 17, which further comprises mixing the sunflower phospholipid composition containing phosphatidylcholine with one or more anionic phospholipids prior to dispersing the mixture in water.

19. The process of any one of claims 17 or 18, which further comprises sterilizing the homogenized emulsion of step iii. by autoclaving.

20. A process for producing a sunflower phospholipid composition containing phosphatidylcholine in an amount of at least 45% by weight, based on the total weight of the sunflower phospholipid composition, and at least one anionic stabilizer compound comprising at least one anionic phospholipid compound and having a content of chlorogenic acids of less than 0.1% by weight, based on the total weight of the sunflower phospholipid composition, where the composition is a mixture of the sunflower phospholipid composition and the anionic stabilizer , which comprises the following steps:
i) extracting a sunflower lecithin with a solvent selected from acetone and mixtures thereof with water, to obtain an extract and a residue, followed by
ii) repeated extraction of the residue with ethanol or a mixture thereof with water to obtain ethanolic extracts,
iii) combining the ethanolic extracts and removing the ethanol and optional water to obtain sunflower phospholipid composition containing phosphatidylcholine in an amount of at least 45% by weight, based on the total weight of the sunflower phospholipid composition, and having a content of chlorogenic acids of less than 0.1% by weight, based on the total weight of the sunflower phospholipid composition,
iv) dissolving the sunflower phospholipid composition obtained in step iii) and at least one further anionic phospholipid compound in an organic solvent and evaporating the solvent.

## Patentansprüche

1. Sonnenblumenphospholipidzusammensetzung, enthaltend Phosphatidylcholin in einer Menge von mindestens 45 Gew.-%, bezogen auf das Gesamtgewicht der Phospholipidzusammensetzung, und weniger als 0,1 Gew.-% Chlorogensäuren, bezogen auf das Gesamtgewicht der Sonnenblumenphospholipidzusammensetzung, in Kombination mit mindestens einer anionischen Stabilisatorverbindung, die mindestens eine anionische Phospholipidverbindung umfasst, als Emulgator in einer wässrigen Öl-in-Wasser-Emulsion zur Verwendung bei der parenteralen Verabreichung.

2. Zusammensetzung nach Anspruch 1, wobei die Sonnenblumenphospholipidzusammensetzung neben dem Phosphatidylcholin mindestens eine anionische Phospholipidverbindung enthält.

3. Zusammensetzung nach Anspruch 1 oder Anspruch 2, wobei die anionische Phospholipidverbindung aus der Gruppe bestehend aus Phosphatidylglycerin, Phosphatidylinositol, PEGyliertem Phosphatidylethanolamin, Phosphatidylserin, Phosphatidsäure, 1,3-Bis(sn-3'-phosphatidyl)-sn-glycerin und Kombinationen davon ausgewählt ist und wobei es sich bei der anionischen Phospholipidverbindung bevorzugt um Phosphatidylglycerin, insbesondere 1,2-Dimyristoyl-sn-glycero-3-phosphorylglycerin handelt.

4. Zusammensetzung nach einem der Ansprüche 1 bis 3, wobei das Gewichtsverhältnis der anionischen Phospholipidverbindung und Sonnenblumenphospholipidzusammensetzung im Bereich von 0,2 : 99,8 bis 20 : 80 liegt.

5. Zusammensetzung nach einem der vorhergehenden Ansprüche, wobei die Sonnenblumenphospholipidzusammensetzung Phosphatidylethanolamin enthält.

6. Zusammensetzung nach einem der vorhergehenden Ansprüche, wobei die Sonnenblumenphospholipidzusammensetzung in einer solchen Menge verwendet wird, dass die Konzentration von Phosphatidylcholin in der Öl-in-Wasser-Emulsion im Bereich von 0,1 bis 20 Gew.-%, bezogen auf das Gesamtgewicht der Wasser-in-Öl-Emulsion, liegt.

7. Zusammensetzung nach einem der vorhergehenden Ansprüche, wobei es sich bei der Öl-in-Wasser-Emulsion zur parenteralen Verabreichung um eine Öl-in-Wasser-Emulsion zur parenteralen Ernährung handelt.

8. Kombination einer Sonnenblumenphospholipidzusammensetzung, die Phosphatidylcholin in einer Menge von mindestens 45 Gew.-%, bezogen auf das Gesamtgewicht
der Sonnenblumenphospholipidzusammensetzung, enthält und einen Gehalt an Chlorogensäuren von weniger als 0,1 Gew.-%, bezogen auf das Gesamtgewicht der Sonnenblumenphospholipidzusammensetzung, aufweist, mit mindestens einer anionischen Stabilisatorverbindung, die mindestens eine anionische Phospholipidverbindung umfasst.

9. Kombination nach Anspruch 8, bei der es sich um eine Sonnenblumenphospholipidzusammensetzung handelt, die Phosphatidylcholin in einer Menge von mindestens 45 Gew.-%, bezogen auf das Gesamtgewicht der Sonnenblumenphospholipidzusammensetzung, und mindestens eine anionische Stabilisatorverbindung, die mindestens eine anionische Phospholipidverbindung umfasst, enthält und einen Gehalt an Chlorogensäuren von weniger als 0,1 Gew.-%, bezogen auf das Gesamtgewicht der Sonnenblumenphospholipidzusammensetzung, aufweist, wobei es sich bei der Zusammensetzung um ein Gemisch der Sonnenblumenphospholipidzusammensetzung und des anionischen Stabilisators handelt.

10. Kombination nach Anspruch 8 oder Anspruch 9, die mindestens eines der folgenden Merkmale 10.a bis 10.b aufweist:
10.a die anionische Phospholipidverbindung ist aus der Gruppe bestehend aus Phosphatidylglycerin, Phosphatidylinositol, PEGyliertem Phosphatidylethanolamin, Phosphatidylserin, Phosphatidsäure, 1,3-Bis(sn-3'-phosphatidyl)-sn-glycerin und Kombinationen davon ausgewählt;
10.b das Gewichtsverhältnis von anionischer Phospholipidverbindung und Phosphatidylcholin liegt im Bereich von 0,2 : 99,8 bis 30 : 70.

11. Wässrige Öl-in-Wasser-Emulsion zur parenteralen Applikation, insbesondere zur parenteralen Ernährung, die Folgendes enthält:
a) eine Ölphase, die eine Triglyceridzusammensetzung umfasst, die zur parenteralen Verabreichung, insbesondere zur parenteralen Ernährung geeignet ist,
b) eine Kombination der Sonnenblumenphospholipidzusammensetzung, die weniger als 0,1 Gew.-% Chlorogensäuren, bezogen auf das Gesamtgewicht der Sonnenblumenphospholipidzusammensetzung, enthält, mit mindestens einer anionischen Stabilisatorverbindung, wobei die Kombination wie in einem der Ansprüche 8 bis 10 definiert ist, und
c) Wasser.

12. Wässrige Öl-in-Wasser-Emulsion nach Anspruch 11, die Folgendes enthält:
a) 5 bis 35 Gew.-%, bezogen auf das Gesamtgewicht der wässrigen Öl-in-Wasser-Emulsion, der Ölphase,
b) die Kombination der Sonnenblumenphospholipidzusammensetzung, die weniger als 0,1 Gew.-% Chlorogensäuren, bezogen auf das Gesamtgewicht der Sonnenblumenphospholipidzusammensetzung, enthält, mit mindestens einer anionischen Phospholipidverbindung, wie in einem der Ansprüche 1 bis 7 definiert,
wobei die Menge der Sonnenblumenzusammensetzung so bemessen ist, dass die Konzentration von Phosphatidylcholin in der Öl-in-Wasser-Emulsion im Bereich von 0,1 bis 20 Gew.-%, bezogen auf das Gesamtgewicht der Öl-in-Wasser-Emulsion, liegt, und
c) Wasser.

13. Wässrige Öl-in-Wasser-Emulsion nach Anspruch 11 oder Anspruch 12, die mindestens eines der Merkmale 13.a oder 13.b aufweist:
13.a sie enthält weniger als 100 ppm Chlorogensäuren;
13.b sie hat einen pH-Wert im Bereich von pH 4 bis pH 10, wie bei 20 °C und 1 bar bestimmt.

14. Wässrige Öl-in-Wasser-Emulsion nach Anspruch 11 oder Anspruch 13, wobei die Ölphase im Wesentlichen aus einer Triglyceridzusammensetzung besteht, die zur parenteralen Verabreichung geeignet ist.

15. Wässrige Öl-in-Wasser-Emulsion nach Anspruch 11 oder Anspruch 14, wobei die anionische Phospholipidverbindung aus der Gruppe bestehend aus Phosphatidylglycerin, Phosphatidylinositol, PEGyliertem Phosphatidylethanolamin, Phosphatidylserin, Phosphatidsäure, 1,3-Bis(sn-3'-phosphatidyl)-sn-glycerin und Kombinationen davon ausgewählt ist.

16. Wässrige Öl-in-Wasser-Emulsion nach Anspruch 11 oder Anspruch 15, wobei das Gewichtsverhältnis von anionischer Phospholipidverbindung und Phosphatidylcholin im Bereich von 0,2 : 99,8 bis 15 : 85 liegt.

17. Verfahren zur Herstellung einer wässrigen Öl-in-Wasser-Emulsion zur parenteralen Verabreichung gemäß einem der Ansprüche 11 bis 16, umfassend
i. Bereitstellen einer wässrigen Phase und einer Ölphase;
ii. Emulgieren der Ölphase in der wässrigen Phase
- in Gegenwart der Kombination der Sonnenblumenphospholipidzusammensetzung, die weniger als 0,1 Gew.-% Chlorogensäuren, bezogen auf das Gesamtgewicht der Sonnenblumenphospholipidzusammensetzung, enthält, mit mindestens einer anionischen Stabilisatorverbindung, die eine anionische Phospholipidverbindung umfasst, wie in einem der Ansprüche 1 bis 7 definiert;
iii. Homogenisieren der in Schritt erhaltenen Emulsion auf eine zur parenteralen Verabreichung geeignete Tröpfchengröße.

18. Verfahren nach Anspruch 17, das ferner Mischen der Phosphatidylcholin enthaltenden Sonnenblumenphospholipidzusammensetzung mit einem oder mehreren anionischen Phospholipiden vor dem Dispergieren des Gemischs in Wasser umfasst.

19. Verfahren nach Anspruch 17 oder Anspruch 18, das ferner Sterilisieren der homogenisierten Emulsion aus Schritt iii. durch Autoklavieren umfasst.

20. Verfahren zur Erzeugung einer Sonnenblumenphospholipidzusammensetzung, die Phosphatidylcholin in einer Menge von mindestens 45 Gew.-%, bezogen auf das Gesamtgewicht der Sonnenblumenphospholipidzusammensetzung, und mindestens eine anionische Stabilisatorverbindung, die mindestens eine anionische Phospholipidverbindung umfasst, enthält und einen Gehalt an Chlorogensäuren von weniger als 0,1 Gew.-%, bezogen auf das Gesamtgewicht der Sonnenblumenphospholipidzusammensetzung, aufweist, wobei es sich bei der Zusammensetzung um ein Gemisch der Sonnenblumenphospholipidzusammensetzung und des anionischen Stabilisators handelt, das die folgenden Schritte umfasst:
i) Extrahieren eines Sonnenblumenlecithins mit einem Lösungsmittel, das aus Aceton und Gemischen davon mit Wasser ausgewählt ist, so dass ein Extrakt und ein Rückstand erhalten werden, gefolgt von
ii) wiederholter Extraktion des Rückstands mit Ethanol oder einem Gemisch davon mit Wasser, so dass ethanolische Extrakte erhalten werden,
iii) Kombinieren der ethanolischen Extrakte und Entfernen des Ethanols und gegebenenfalls des Wassers, so dass eine Sonnenblumenphospholipidzusammensetzung erhalten wird, die Phosphatidylcholin in einer Menge von mindestens 45 Gew.-%, bezogen auf das Gesamtgewicht der Sonnenblumenphospholipidzusammensetzung, enthält und einen Gehalt an Chlorogensäuren von weniger als 0,1 Gew.-%, bezogen auf das Gesamtgewicht der Sonnenblumenphospholipidzusammensetzung, aufweist,
iv) Lösen der in Schritt iii) erhaltenen Sonnenblumenphospholipidzusammensetzung und mindestens einer weiteren anionischen Phospholipidverbindung in einem organischen Lösungsmittel und Abdampfen des Lösungsmittels.

## Revendications

1. Composition de phospholipide de tournesol contenant de la phosphatidylcholine en une quantité d'au moins 45 % en poids, sur la base du poids total de la composition de phospholipide, et contenant moins de 0,1 % en poids d'acides chlorogéniques, sur la base du poids total de la composition de phospholipide de tournesol, en combinaison avec au moins un composé stabilisant anionique comprenant au moins un composé de phospholipide anionique en tant qu'émulsifiant dans une émulsion aqueuse huile-dans-eau destinée à l'administration parentérale.

2. Composition selon la revendication 1, dans laquelle la composition de phospholipide de tournesol contient au moins un composé de phospholipide anionique en plus de la phosphatidylcholine.

3. Composition selon l'une quelconque des revendications 1 ou 2, dans laquelle le composé de phospholipide anionique est choisi dans le groupe constitué par phosphatidylglycérol, phosphatidylinositol, phosphatidyléthanolamine PEGylée, phosphatidylsérine, acide phosphatidique, 1,3-bis(sn-3'-phosphatidyl)-sn-glycérol et leurs combinaisons, et dans laquelle le composé de phospholipide anionique est de préférence le phosphatidylglycérol, en particulier le 1,2-dimyristoyl-sn-glycéro-3-phosphorylglycérol.

4. Composition selon l'une quelconque des revendications 1 à 3, dans laquelle le rapport en poids du composé de phospholipide anionique et de la composition de phospholipide de tournesol est dans la plage de 0,2 : 99,8 à 20 : 80.

5. Composition selon l'une quelconque des revendications précédentes, dans laquelle la composition de phospholipide de tournesol contient de la phosphatidyléthanolamine.

6. Composition selon l'une quelconque des revendications précédentes, dans laquelle la composition de phospholipide de tournesol est utilisée en une quantité telle que la concentration de phosphatidylcholine dans l'émulsion huile-dans-eau est dans la plage de 0,1 à 20 % en poids, sur la base du poids total de l'émulsion eau-dans-huile.

7. Composition selon l'une quelconque des revendications précédentes, dans laquelle l'émulsion huile-dans-eau pour administration parentérale est une émulsion huile-dans-eau pour nutrition parentérale.

8. Combinaison d'une composition de phospholipide de tournesol contenant de la phosphatidylcholine en une quantité d'au moins 45 % en poids, sur la base du poids total de la composition de phospholipide de tournesol et ayant une teneur en acides chlorogéniques inférieure à 0,1 % en poids, sur la base du poids total de la composition de phospholipide de tournesol, avec au moins un composé stabilisant anionique comprenant au moins un composé de phospholipide anionique.

9. Combinaison selon la revendication 8, qui est une composition de phospholipide de tournesol contenant de la phosphatidylcholine en une quantité d'au moins 45 % en poids, sur la base du poids total de la composition de phospholipide de tournesol, et au moins un composé stabilisant anionique comprenant au moins un composé de phospholipide anionique et ayant une teneur en acides chlorogéniques inférieure à 0,1 % en poids, sur la base du poids total de la composition de phospholipide de tournesol, la composition étant un mélange de la composition de phospholipide de tournesol et du stabilisant anionique.

10. Combinaison selon la revendication 8 ou la revendication 9, qui présente au moins l'une des caractéristiques suivantes 10.a à 10.b :
10.a le composé de phospholipide anionique est choisi dans le groupe constitué par phosphatidylglycérol, phosphatidylinositol, phosphatidyléthanolamine PEGylée, phosphatidylsérine, acide phosphatidique, 1,3-bis(sn-3'-phosphatidyl)-sn-glycérol et leurs combinaisons ;
10.b le rapport en poids du composé de phospholipide anionique et de la phosphatidylcholine est dans la plage de 0,2 : 99,8 à 30 : 70.

11. Émulsion aqueuse huile-dans-eau pour administration parentérale, en particulier pour nutrition parentérale qui contient
a) une phase huileuse comprenant une composition de triglycérides appropriée pour une administration parentérale, en particulier pour une nutrition parentérale,
b) une combinaison de la composition de phospholipide de tournesol contenant moins de 0,1 % en poids d'acides chlorogéniques, sur la base du poids total de la composition de phospholipide de tournesol, avec au moins un composé stabilisant anionique, la combinaison étant telle que définie dans l'une quelconque des revendications 8 à 10, et
c) de l'eau.

12. Émulsion aqueuse huile-dans-eau selon la revendication 11, qui contient
a) 5 à 35 % en poids, sur la base du poids total de l'émulsion aqueuse huile-dans-eau, de la phase huileuse,
b) la combinaison de la composition de phospholipide de tournesol contenant moins de 0,1 % en poids d'acides chlorogéniques, sur la base du poids total de la composition de phospholipide de tournesol, avec au moins un composé de phospholipide anionique, tel que défini dans l'une quelconque des revendications 1 à 7,
dans laquelle la quantité de la composition de tournesol est telle que la concentration de phosphatidylcholine dans l'émulsion huile-dans-eau est dans la plage de 0,1 à 20 % en poids, sur la base du poids total de l'émulsion huile-dans-eau, et
c) de l'eau.

13. Émulsion aqueuse huile-dans-eau selon l'une quelconque des revendications 11 ou 12, qui présente au moins l'une des caractéristiques 13.a ou 13.b :
13.a elle contient moins de 100 ppm d'acides chlorogéniques ;
13.b elle a une valeur de pH dans la plage de pH 4 à pH 10, comme déterminée à 20 °C et 1 bar.

14. Émulsion aqueuse huile-dans-eau selon l'une quelconque des revendications 11 ou 13, dans laquelle la phase huileuse est essentiellement constituée d'une composition de triglycérides appropriée pour une administration parentérale.

15. Émulsion aqueuse huile-dans-eau selon l'une quelconque des revendications 11 ou 14, dans laquelle le composé de phospholipide anionique est choisi dans le groupe constitué par phosphatidylglycérol, phosphatidylinositol, phosphatidyléthanolamine PEGylée, phosphatidylsérine, acide phosphatidique, 1,3-bis(sn-3'-phosphatidyl)-sn-glycérol et leurs combinaisons.

16. Émulsion aqueuse huile-dans-eau selon l'une quelconque des revendications 11 ou 15, dans laquelle le rapport en poids du composé de phospholipide anionique et de la phosphatidylcholine est dans la plage de 0,2 : 99,8 à 15 : 85.

17. Procédé de préparation d'une émulsion aqueuse huile-dans-eau pour administration parentérale selon l'une quelconque des revendications 11 à 16, comprenant
i. fourniture d'une phase aqueuse et d'une phase huileuse ;
ii. émulsification de la phase huileuse dans la phase aqueuse
- en présence de la combinaison de la composition de phospholipide de tournesol contenant moins de 0,1 % en poids d'acides chlorogéniques, sur la base du poids total de la composition de phospholipide de tournesol, avec au moins un composé stabilisant anionique comprenant un composé de phospholipide anionique, tel que défini dans l'une quelconque des revendications 1 à 7 ;
iii. homogénéisation de l'émulsion obtenue à l'étape jusqu'à une taille de gouttelette appropriée pour une administration parentérale.

18. Procédé selon la revendication 17, qui comprend en outre le mélange de la composition de phospholipide de tournesol contenant de la phosphatidylcholine avec un ou plusieurs phospholipides anioniques avant la dispersion du mélange dans l'eau.

19. Procédé selon l'une quelconque des revendications 17 ou 18, qui comprend en outre la stérilisation de l'émulsion homogénéisée de l'étape iii. par autoclavage.

20. Procédé de production d'une composition de phospholipide de tournesol contenant de la phosphatidylcholine en une quantité d'au moins 45 % en poids, sur la base du poids total de la composition de phospholipide de tournesol, et au moins un composé stabilisant anionique comprenant au moins un composé de phospholipide anionique et ayant une teneur en acides chlorogéniques inférieure à 0,1 % en poids, sur base du poids total de la composition de phospholipide de tournesol, la composition étant un mélange de la composition de phospholipide de tournesol et du stabilisant anionique, qui comprend les étapes suivantes :
i) extraction d'une lécithine de tournesol avec un solvant choisi parmi l'acétone et des mélanges de celle-ci avec de l'eau, pour obtenir un extrait et un résidu, suivie par
ii) extraction répétée du résidu avec de l'éthanol ou un mélange de celui-ci avec de l'eau pour obtenir des extraits éthanoliques,
iii) combinaison des extraits éthanoliques et élimination de l'éthanol et d'eau éventuelle pour obtenir une composition de phospholipide de tournesol contenant de la phosphatidylcholine en une quantité d'au moins 45 % en poids, sur la base du poids total de la composition de phospholipide de tournesol, et ayant une teneur en acides chlorogéniques inférieure à 0,1 % en poids, sur la base du poids total de la composition de phospholipide de tournesol,
iv) dissolution de la composition de phospholipide de tournesol obtenue à l'étape iii) et d'au moins un autre composé de phospholipide anionique dans un solvant organique et évaporation du solvant.
